# EUROPÄISCHE PATENTSCHRIFT

(11) **EP 3 589 737 B1**
(45) Veröffentlichungstag und Bekanntmachung des Hinweises auf die Patenterteilung: **30.10.2024**
(21) Anmeldenummer: 18708396.9
(22) Anmeldetag: 28.02.2018
(51) Int. Cl.: C12N 15/82, A01H 1/08, C12N 9/18, C07K 14/415

(54) **HAPLOIDISIERUNG IN SORGHUM**
HAPLOIDIZATION IN SORGHUM
HAPLOIDISATION DANS LE SORGHO

(30) Priorität: 28.02.2017 EP 17158439
(43) Veröffentlichungstag der Anmeldung: 08.01.2020
(73) Patentinhaber: KWS SAAT SE & Co. KGaA, 37574 Einbeck (DE)
(72) Erfinder: KLOIBER-MAITZ, Monika, 37574 Einbeck (DE); WIECKHORST, Silke, 37574 Einbeck (DE); BOLDUAN, Christof, 37574 Einbeck (DE); OUZUNOVA, Milena, 37077 Göttingen (DE)
(86) Internationale Anmeldenummer: PCT/EP2018/054901
(87) Internationale Veröffentlichungsnummer: WO 2018/158301

(56) Entgegenhaltungen:
- WO-A1-2016/075255
- WO-A1-2016/177887
- LAURINE M GILLES ET AL: "Loss of pollen-specific phospholipase NOT LIKE DAD triggers gynogenesis in maize", THE EMBO JOURNAL 17 DEC 2014, vol. 36, no. 6, 22 February 2017 (2017-02-22), pages 707 - 717, XP055376375, ISSN: 1460-2075, DOI: 10.15252/embj.201796603
- TIMOTHY KELLIHER ET AL: "MATRILINEAL, a sperm-specific phospholipase, triggers maize haploid induction", NATURE, vol. 542, no. 7639, 23 January 2017 (2017-01-23), United Kingdom, pages 105 - 109, XP055376380, ISSN: 0028-0836, DOI: 10.1038/nature20827
- CHENXU LIU ET AL: "A 4-bp Insertion at ZmPLA1 Encoding a Putative Phospholipase A Generates Haploid Induction in Maize", MOLECULAR PLANT, vol. 10, no. 3, 4 February 2017 (2017-02-04), pages 520 - 522, XP055376385, DOI: 10.1016/j.molp.2017.01.011

## Beschreibung

### GEBIET DER ERFINDUNG

Die vorliegende Erfindung betrifft das Gebiet der Vereinfachung arbeitsaufwendiger Züchtungsprogramme mittels molekularbiologischer Methoden, Markertechnologie sowie der Gentechnik. Insbesondere werden Sorghumhirsepflanzen zur Verfügung gestellt, die durch Modifikationen im Genom, die eine vorzugsweise pollenspezifisch exprimierte Patatin-Phospholipase betreffen, in der Lage sind Haploidie zu induzieren, wodurch haploide Nachkommen erzeugt und in kurzer Zeit durch Chromosomenverdopplung Inzuchtlinien für die Hybridzüchtung hergestellt werden können. Im Speziellen werden Sorghumhirsepflanzen zur Verfügung gestellt, die Mutationen in der Patatin-Phospholipase aufweisen, Verfahren zur Herstellung und Identifizierung dieser Mutationen bzw. der mutierten Pflanze sowie das entsprechende Nukleinsäuremolekül, welches die mutierte Patatin-Phospholipase kodiert sowie Vektoren und Wirtszellen insbesondere Pflanzenzellen enthaltend das Nukleinsäuremolekül, und aus solchen Pflanzenzellen generierte Pflanzen, die in der Lage sind Haploidie zu erzeugen sowie deren Nachkommen, Kreuzungsprodukte, Inzuchtlinien und jeweils deren Pflanzenteile und -produkte.

Basierend auf den Erkenntnissen aus Sorghum stellt die vorliegende Erfindung Verfahren zur Herstellung und Identifizierung von transgenen und nicht-transgenen pflanzlichen Haploiden-Induktoren bereit sowie die entsprechenden Pflanzen, die die Eigenschaft der Haploidie-Induktion erhalten haben oder dessen Induktionsleistung verbessert wurde. Ferner sind von der Erfindung auch Samen oder Nachkommen, Organe, Pflanzenteile, Gewebe oder Zellen der erfindungsgemäßen Pflanze sowie deren Verwendung miterfasst.

### HINTERGRUND DER ERFINDUNG

Sorghum ist eine in Deutschland recht neue Kultur, gerät jedoch aufgrund ihrer Massenwüchsigkeit, Trockentoleranz sowie gute Wasser- und Nährstoffeffizienz in den Fokus der Wissenschaft und Praxis bei der Suche nach weiteren hochertragsreichen Kulturen zur Substratproduktion, insbesondere im Hinblick auf die Verwendung in der Biogasproduktion, aber auch zur Verwendung als Futtermittel, Nahrungsmittel und zur Ethanol-Produktion. Allgemeine Zuchtziele für Sorghum sind die Anpassung an das Klima Mitteleuropas, d.h. eine verbesserte Kältetoleranz, die Verbesserung des Ertrags, der Standfestigkeit gepaart mit guter Jugendentwicklung sowie Entwicklung von Krankheits- und Schädlingsresistenzen.

Sorghum kann wie eine selbstbefruchtende Kultur züchterisch verbessert werden. Die Verbesserung der Population kann durch die aufwendige Selektionszüchtung durch eine gezielte Auswahl der besten Pflanzen zur Saatgutproduktion für das nächste Anbaujahr erreicht werden. Daneben wurden mittels der Hybridzüchtung neue Möglichkeiten zur Verbesserung der Sorten geschaffen. Allerdings beruht der Prozess der Hybridzüchtung auf eine mehrere Generationen dauernde Erzeugung von reinerbigen Vater- und Mutterlinien, wodurch auch der Prozess der Hybridzüchtung trotz guter Erfolge zeit- und kostenintensiv ist.

In WO2016/177887A1 ist ein isoliertes Polypeptid beschrieben, das für die Haploideninduktion in Maispflanzen verantwortlich ist.

WO2016/075255A1 betrifft die Bereitstellung von technischen Mitteln wie Nukleinsäuren, welche nach Transkription oder nach Expression in einer Pflanze geeignet sind, die Eigenschaft eines Haploideninduktors zu vermitteln oder die Induktionsleistung eines Haploideninduktors zu erhöhen.

Gilles et al. (2014) beschreibt, dass der Verlust der pollenspezifischen Phospholipase NOT LIKE DAD Gynogenese in Mais auslöst.

In Kelliher et al. (2017) ist die Induktion von Haploiden in Mais durch die Phospholipase MATRII,INEAL beschrieben.

Liu et al. (2017) betrifft die Haploideninduktion in Mais durch eine 4-bp Insertion in ZmPLA1, die eine putative Phospholipase A kodiert.

Aufgabe der vorliegenden Erfindung war es daher ein effizientes System zur Züchtung von Sorghum bereitzustellen.

### ZUSAMMENFASSUNG DER ERFINDUNG

Die vorliegende Erfindung betrifft das Gebiet der Vereinfachung von arbeitsaufwendigen Züchtungsprogrammen, der Markertechnologie sowie der Gentechnik. Die Erfindung stellt Sorghumhirsepflanzen zur Verfügung, die durch Modifikationen im Genom, die eine pollenspezifisch exprimierte Patatin-Phospholipase betreffen, in der Lage sind Haploidie zu induzieren, wodurch haploide Nachkommen erzeugt und in kurzer Zeit durch Chromosomenverdopplung Inzuchtlinien, d.h. reinerbige Vater- und Mutterlinien, für die Hybridzüchtung hergestellt werden können. Zusätzlich können die Erkenntnisse dazu verwendet werden, transgene sowie nicht-transgene pflanzliche Haploiden-Induktoren herzustellen oder die Induktionsleistung von Pflanzen zu verbessern.

Zunächst werden einige der in dieser Anmeldung verwendeten Begriffe nachfolgend näher erläutert:
"Die Eigenschaft eines Haploideninduktors vermitteln" oder das "Vermitteln der Eigenschaft eines Haploideninduktors" oder "in der Lage sein Haploidie zu induzieren" ein vergleichbarer Ausdruck bedeutet, dass eine Pflanze durch Verwendung einer erfindungsgemäßen Nukleinsäure bzw. durch Modifizierung des Genoms, im Speziellen durch Mutation einer Patatin-Phospholipase, in die Lage versetzt wird, befruchtete Samen oder Embryos, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Eigenschaft eines Haploideninduktors, angegeben als absolute Haploiden-Induktionsrate, meint, dass mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, besonders bevorzugt mindestens 6%, 7%, 8%, 9%, 10%, 11 %, 12%, 13%, 14% oder 15%, oder ganz besonders bevorzugt mindestens 20%, 25%, 30%, 35%, 40%, 45% oder 50% der befruchteten Samen oder Embryos einen haploiden Chromosomensatz aufweisen.

Ein "funktionelles Fragment" einer Nukleotidsequenz meint einen Abschnitt einer Nukleotidsequenz, welcher die identische oder eine vergleichbare Funktionalität wie die Gesamtnukleotidsequenz, aus welcher das funktionelle Fragment stammt, aufweist. Als solches kann das funktionelle Fragment eine Nukleotidsequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit der Gesamtnukleotidsequenz ist. Weiterhin kann ein "funktionelles Fragment" einer Nukleotidsequenz auch einen Abschnitt einer Nukleotidsequenz meinen, welcher die Funktionalität der Gesamtnukleotidsequenz beispielsweise im Zuge von posttranskriptionalem oder transkriptionellem Gene Silencing verändert. Als solches kann das funktionelle Fragment einer Nukleotidsequenz mindestens 14, 15, 16, 17, 18, 19, 20, 21, 22, 23, 24 oder 25, bevorzugt mindestens 30, 35, 40, 45, 50, 60, 70, 80, 90, 100, 120 oder 140, besonders bevorzugt mindestens 160, 180, 200, 250, 300, 350, 400, 450, 500, 600, 700, 800, 900 oder 1000 aufeinanderfolgende Nukleotide der Gesamtnukleotidsequenz umfassen.

Ein "funktioneller Teil" eines Proteins meint einen Abschnitt eines Proteins bzw. eine Anschnitt der Aminosäuresequenz, die das Protein kodiert, wobei der Abschnitt die identische oder eine vergleichbare Funktionalität wie das Gesamtprotein in einer pflanzlichen Zelle ausüben kann. Ein funktioneller Teil eines Proteins weist über eine Länge von mindestens 50%, 55%, 60%, 65%, 70%, 75%, 80%, 85%, 90%, 92%, 94% 96%, 97%, 98% oder 99% eine identische oder unter Berücksichtigung von konservativen und semikonservativen Aminosäureaustauschen ähnliche Aminosäuresequenz auf wie das Protein, aus welchem der funktionelle Teil stammt.

"Haploideninduktor" meint auch einen *in vivo* Haploideninduktor.

Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d. h. Basenpaarungen mit diesem eingeht. Standardverfahren zur Hybridisierung sind beispielsweise in Sambrook et al., Molecular Cloning: A Laboratory Manual 3rd Ed. Cold Spring Harbor Laboratory Press, Cold Spring Harbor, NY, 2001 beschrieben. Vorzugsweise wird darunter verstanden, dass mindestens 80% oder 85%, bevorzugt mindestens 90%, 91%, 92%, 93%, 94% oder 95%, besonders bevorzugt mindestens 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen und Homologen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 80%, mindestens 85%, mindestens 90% oder mindestens 95% Sequenzidentität aufweisen. Stringente Hybridisierungsbedingungen sind beispielsweise: Hybridisieren in 4 × SSC bei 65°C und anschließendes mehrfaches Waschen in 0,1 × SSC bei 65°C für insgesamt etwa 1 Stunde. Der hier verwendete Begriff "stringente Hybridisierungsbedingungen" kann auch bedeuten: Hybridisieren bei 68°C in 0,25 M Natriumphosphat, pH 7,2, 7% SDS, 1 mM EDTA und 1% BSA für 16 Stunden und anschließendes zweimaliges Waschen mit 2 × SSC und 0,1% SDS bei 68°C. Bevorzugt findet eine Hybridisierung unter stringenten Bedingungen statt.

"Die Induktionsleistung eines Haploideninduktors erhöhen" oder "die Erhöhung der Induktionsleistung eines Haploideninduktors" oder vergleichbare Ausdrücke meinen, dass die Haploiden-Induktionsrate einer Pflanze, welche die Eigenschaft eines Haploideninduktors aufweist, erhöht wird. Somit kann die Zahl der befruchteten Samen, welche einen haploiden Chromosomensatz aufweisen und aus einer Kreuzung des Haploideninduktors mit einer Pflanze derselben Gattung, vorzugsweise derselben Spezies, welche nicht die Eigenschaft eines Haploideninduktors aufweist, hervorgegangen sind, um mindestens 0,1 %, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% höher sind als die Zahl der haploiden befruchteten Samen, welche ohne die Verwendung der Nukleinsäure bzw. ohne Modifizierung des Genoms, im Speziellen ohne Mutation einer Patatin-Phospholipase im Sinne der vorliegenden Erfindung erzielt wird, d.h. die Haploiden-Induktionsrate kann um mindestens 0,1 %, 0,2%, 0,3%, 0,4%, 0,5%, 0,6%, 0,7%, 0,8%, 0,9% oder 1 %, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% relativ zu der zuvor erreichten Haploiden-Induktionsrate gesteigert werden.

"Komplementäre" Nukleotidsequenz bedeutet bezogen auf eine Nukleinsäure in Form einer doppelsträngigen DNA, dass der zum ersten DNA Strang komplementäre zweite DNA Strang entsprechend den Basenpaarungsregeln die Nukleotide aufweist, die zu den Basen des ersten Stranges entsprechend den Watson-Crick-Regeln korrespondieren.

Ein "molekularer Marker" ist eine Nukleinsäure, die polymorph in einer pflanzlichen Population ist und als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Somit ist ein solcher Marker in der Lage verschiedene allelische Zustände (Allele) zu detektieren und zu unterscheiden. Der Begriff "molekularer Marker" bezieht sich auch auf Nukleotidsequenzen, welche komplementär oder zumindest weitestgehend komplementär oder homolog sind zu genomischen Sequenzen, zum Beispiel Nukleinsäuren, welche als Sonden oder Primer eingesetzt werden. Für Marker finden sich diese Unterschiede auf DNA-Ebene und sind beispielsweise Polynukleotidsequenzunterschiede wie zum Beispiel SSRs (*simple sequence repeats*), RFLPs (*restriction fragment length polymorphisms*), FLPs (*fragment length polymorphisms*) oder SNPs (*single nucleotide polymorphisms*)*.* Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, die genetische Polymorphismen (zwischen Teilen einer Population) beschreiben, können unter Verwendung gut etablierter Verfahren aus dem Stand der Technik nachgewiesen werden (An Introduction to Genetic Analysis. 7th Edition, Griffiths, Miller, Suzuki et al., 2000). Dazu gehören z.B. DNA-Sequenzierung, PCR-basierte sequenzspezifische Amplifikation, Nachweis von RFLPs, Nachweis von Polynukleotid-Polymorphismen mittels Allel-spezifischer Hybridisierung (ASH), Detektion von amplifizierten variablen Sequenzen des Pflanzengenoms, Detektion einer 3SR (*self-sustained sequence replication*), Detektion von SSRs, SNPs, RFLPs oder AFLPs (*amplified fragment length polymorphisms*). Ferner sind auch die Methoden zur Detektion von ESTs (*expressed sequence tags*) und SSR-Marker abgeleitet von EST-Sequenzen und RAPD (*randomly amplifiedpolymorphic DNA*) bekannt. Je nach Kontext kann der Begriff Marker in der Beschreibung auch eine spezifische Chromosom-Position in dem Genom einer Spezies, wo ein spezifischer Marker (z.B. SNP) gefunden werden kann, meinen.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors

Eine "Pflanze" im Sinne der Erfindung kann, sofern nicht anders angegeben, von jeder Spezies aus den dikotyledonen und monokotyledonen Pflanzen sein. Bevorzugt sind Pflanzen in Agrikultur oder Hortikultur oder zur Erzeugung von Bioenergie (Bioethanol, Biogas etc.). Hierzu zählen beispielhaft *Solanum tuberosum*, *Triticum aestivum*, *Triticum durum*, *Triticum spelta, Helianthus annuus, Secale cereale, Hordeum vulgare, Hordeum bulbosum, Brassica napus, Brassica oleracea, Brassica rapa, Brassica juncacea, Brassica nigra, Glycine max, Gossypium sp., Sorghum bicolor, Sorghum sudanense, Sorghum bicolor* × *Sorghum sudanense,* Triticale, *Saccharum officinarium*, *Setaria italica, Oryza sativa, Oryza minuta, Oryza australiensis, Oryza alta, Brachypodium distachyon, Hordeum marinum, Aegilops tauschii, Daucus glochidiatus, Daucus pusillus, Daucus muricatus, Daucus carota, Eucalyptus grandis, Erythranthe guttata, Genlisea aurea, Musa sp., Avena sp., Nicotiana sylvestris, Nicotiana tabacum, Nicotiana tomentosiformis*, *Solanum lycopersicum, Coffea canephora, Vitis vinifera, Cucumis sativus, Morus notabilis, Crucihimalaya himalaica, Crucihimalaya wallichii, Cardamine flexuosa, Lepidium virginicum, Capsella bursa-pastoris, Olmarabidopsis pumila, Arabis hirsuta, Raphanus sativus, Eruca vesicaria sativa, Citrus sinensis, Jatropha curcas, Populus trichocarpa* oder *Beta vulgaris.*

Eine erfindungsgemäße Sorghumhirsepflanze ist eine Pflanze der Gattung *Sorghum*, insbesondere der Spezies *Sorghum bicolor, Sorghum sudanense, Sorghum bicolor* × *Sorghum sudanense, Sorghum* × *almum* (*Sorghum bicolor* × *Sorghum halepense*), Sorghum arundinaceum, Sorghum × drummondii, *Sorghum halepense* und/oder Sorghum propinquum bzw. deren Hybriden sowie sämtliche sich davon ableitenden Sorten.

Pflanzliche "Organe" meinen beispielsweise Blätter, Sprossachse, Stamm, Wurzeln, vegetative Knospen, Meristeme, Embryos, Antheren, Ovula, Samen oder Früchte, insbesondere Samenkörner. Der Begriff "Pflanzenteil" bzw. "Pflanzenteile" beinhaltet, ist jedoch nicht beschränkt auf, die Sprossachse bzw. den Halm, Blätter, Blüten, Blütenstände, Wurzeln, Früchte und Samen sowie die Pollen. Pflanzliche "Teile" meinen ferner einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe sowie das Bildungsgewebe, Grundgewebe (das sogenannte Parenchym), Leitgewebe, Festigungsgewebe und das Deckgewebe (die sogenannte Epidermis). Das Gewebe wird durch diese Auflistung jedoch nicht beschränkt. Unter pflanzlichen "Zellen" sind beispielsweise isolierte Zellen mit einer Zellwand oder Aggregate davon oder Protoplasten zu verstehen.

Ein "Promotor" ist ein nicht-translatierter DNA-Abschnitt, typischerweise stromaufwärts einer kodierenden Region, welche die Bindestelle für die RNA-Polymerase beinhaltet und die Transkription der DNA initiiert. Ein Promotor enthält zudem andere Elemente, die als Regulatorgen der Genexpression fungieren (z.B. cis-regulatorischen Elemente). Ein "Kern-oder Minimalpromotor" ist ein Promoter, der zumindest die Grundelemente, welche für die Transkriptionsinitiation gebraucht werden, aufweist (z.B. TATA-Box und/oder Initiator).

Im Zusammenhang mit der vorliegenden Erfindung betrifft der Begriff "Modifikationen" eine Nukleotidsequenz, welche die Spezifität und/oder die Expressionsstarke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Eine "Transgene Pflanze" bezieht sich auf eine Pflanze, in deren Genom mindestens ein Polynukleotid, vorzugsweise ein heterologes Polynukleotid, integriert ist. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

"Zur Verwendung als Haploideninduktor geeignet" oder "ist in der Lage Haploidie zu induzieren" meint, dass eine Pflanze in die Lage ist, befruchtete Samen, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Verwendung als Haploideninduktor, angegeben als absolute Haploiden-Induktionsrate, meint, dass mindestens 0,1 %, 0,2%, 0,3%, 0,4%, 0,5%, 0,6%, 0,7%, 0,8%, 0,9% oder 1 %, bevorzugt mindestens 1,5%, 2%, 2,5%, 3%, 3,5%, 4%, 4,5% oder 5%, besonders bevorzugt mindestens 6%, 7%, 8%, 9%, 10%, 11 %, 12%, 13%, 14% oder 15%, oder ganz besonders bevorzugt mindestens 20%, 25%, 30%, 35%, 40%, 45% oder 50% der befruchteten Samen einen haploiden Chromosomensatz aufweisen.

Ausgestaltungen und Ausführungsformen der vorliegenden Beschreibung werden in exemplarischer Weise mit Bezug auf die angehängten Figuren und Sequenzen beschrieben:

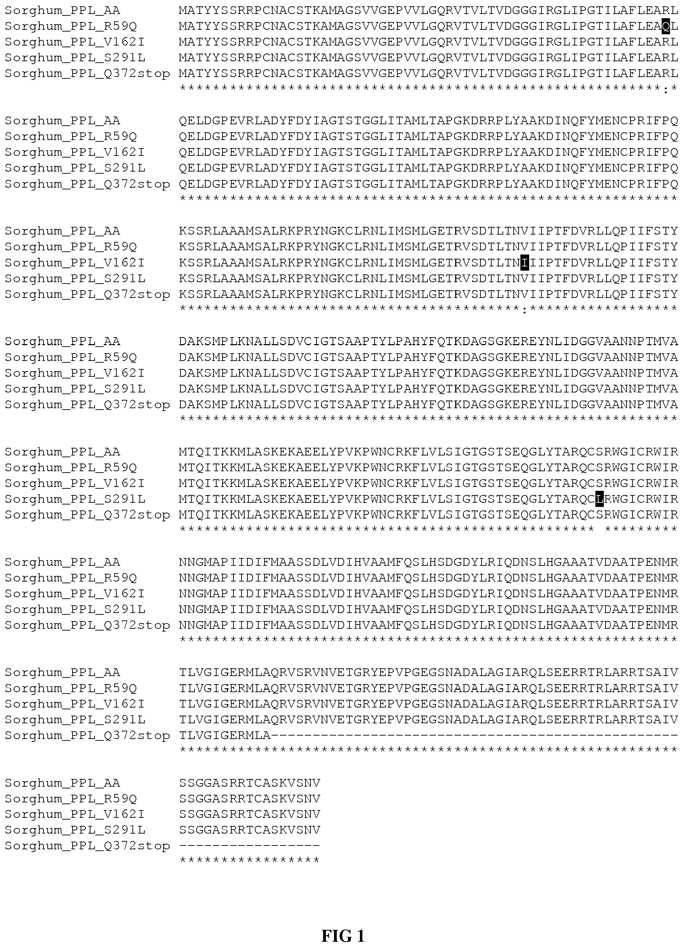

- **Figur 1:**: Sequenzalignment (CLUSTAL O 1.2.4) der Aminosäuresequenzen der Wild-Typ Patatin-Phospholipase aus Sorghumhirse (Sorghum_PPL_AA, SEQ ID Nr.: 3), der Patatin-Phospholipase aus Sorghumhirse mit einem Austausch der Aminosäure Arginin (R) durch Glutamin (Q) an der Aminosäureposition 59 (Sorghum_PPL_R59Q, SEQ ID Nr.: 6), der Patatin-Phospholipase aus Sorghumhirse mit einem Austausch der Aminosäure Valin (V) durch Isoleucin (I) an der Aminosäureposition 162 (Sorghum_PPL_V162I, SEQ ID Nr.: 9), der Patatin-Phospholipase aus Sorghumhirse mit einem Austausch der Aminosäure Serin (S) durch Leucin (L) an der Aminosäureposition 291 (Sorghum _PPL_S291L, SEQ ID Nr.: 12) und der Patatin-Phospholipase aus Sorghumhirse mit einem Austausch der Aminosäure Glutamin (Q) durch ein Stopp-Codon an der Aminosäureposition 372 (Sorghum _PPL_Q372Stopp, SEQ ID Nr.: 15). Die Aminosäurepositionen 59, 162 und 291 sind schwarz unterlegt.

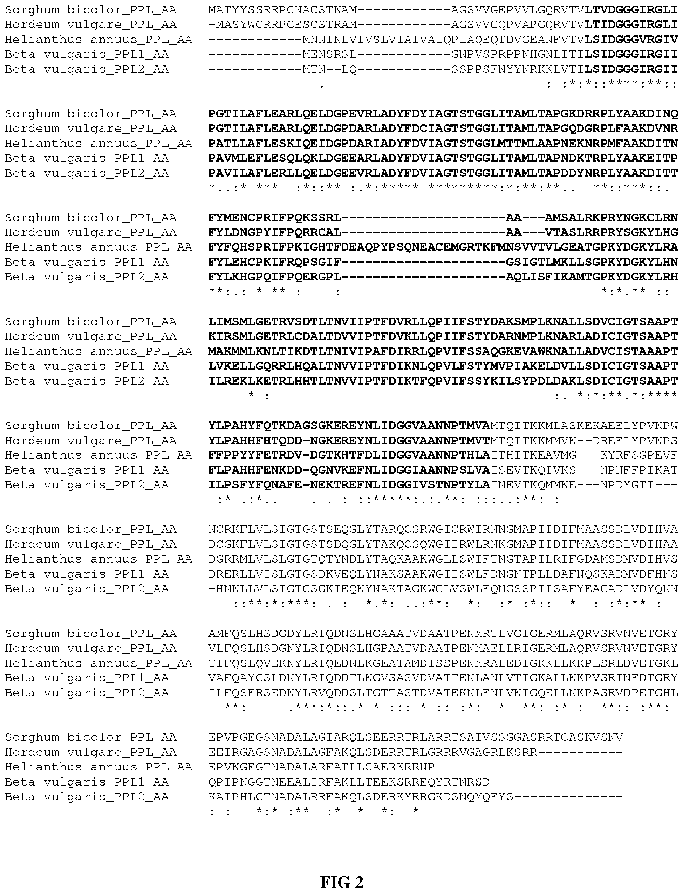

- **Figur 2:**: Sequenzalignment (CLUSTAL O 1.2.4) der Aminosäuresequenzen der Wild-Typ Patatin-Phospholipase aus Sorghumhirse (Sorghum bicolor_PPL_AA, SEQ ID Nr.: 3), der Patatin-Phospholipase aus Gerste (Hordeum vulgare_PPL_AA, SEQ ID Nr.: 21), der Patatin-Phospholipase aus Sonnenblume (Helianthus annuus_PPL_AA, SEQ ID Nr.: 18), der Patatin-Phospholipase 1 aus Zuckerrübe (Beta vulgaris_PPL1_AA, SEQ ID Nr.: 24) und der Patatin-Phospholipase 2 aus Zuckerrübe (Beta vulgaris _Phospholipase2_AA, SEQ ID Nr.: 27). Die Aminosäurepositionen, welche die funktionelle Domäne der Phospholipase darstellen, sind fett gedruckt.

### AUSFÜHRLICHE BESCHREIBUNG DER ERFINDUNG

Die vorliegende Erfindung stellt eine Pflanze der Gattung Sorghum, im Folgenden auch als Sorghumhirsepflanze bezeichnet, zur Verfügung, die in der Lage ist Haploidie zu induzieren. Durch diese Eigenschaft der Haploideninduktion ist die Pflanze in der Lage, befruchtete Samen oder Embryos, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Durch dieses System war es den Erfindern möglich, ein effizientes System zur Züchtung von Sorghumhirsepflanzen bereitzustellen, denn durch Chromosomenverdopplung in den haploiden Nachkommen können Inzuchtlinien, d.h. reinerbige Vater- und Mutterlinien für die Hybridzüchtung generiert werden.

Die erfindungsgemäßen Sorghumhirsepflanzen sind dadurch gekennzeichnet, dass sie eine oder mehrere Modifikationen aufweisen, die die Patatin-Phospholipase betreffen, wodurch entweder die Eigenschaft zur Haploideninduktion vermittelt wird oder auch eine natürlich vorhandenen Fähigkeit zur Haploideninduktion verbessert bzw. die Induktionsleistung erhöht wird. Somit ist die Modifikation betreffend die Patatin-Phospholipase ursächlich für die Eignung der erfindungsgemäßen Sorghumhirsepflanze als Haploideninduktor in Züchtungsprogrammen.

Die Pflanzen zeichnen sich dadurch aus, dass sie eine Induktionsrate von vorzugsweise mindestens 0,5% aufweisen und sich somit von Wildtyp-Pflanzen bzw. von Nicht-Haploideninduktoren unterscheiden.

In den im Rahmen der vorliegenden Erfindung durchgeführten Experimenten wurde festgestellt, dass die Patatin-Phospholipase in Sorghumhirse pollenspezifisch exprimiert wird und daher vermutlich einen Einfluss auf das Pollenschlauch-Wachstum oder die Interaktion zwischen den Gametophyten hat. Somit ist die Sorghumhirsepflanze vorzugsweise dadurch gekennzeichnet, dass die Patatin-Phospholipase pollenspezifisch exprimiert wird und/oder Einfluss auf das Pollenschlauch-Wachstum oder die Interaktion zwischen den Gametophyten hat.

Die Patatin-Phospholipase ist durch die Nukleotidsequenz gemäß SEQ ID Nr.: 1 oder 2 kodiert oder durch eine Nukleotidsequenz, die zu mindestens 80%, vorzugsweise 85%, weiter bevorzugt 90%, noch weiter bevorzugt 95% und am bevorzugtesten 99% identisch zu SEQ ID Nr.: 1 oder 2 ist, kodiert ist oder durch eine Nukleotidsequenz kodiert wird, die mit der komplementären Sequenz zu der Nukleotidsequenz gemäß SEQ ID Nr.: 1 oder 2 unter stringenten Bedingungen hybridisiert, wobei die stringenten Bedingungen Hybridisieren in 4 × SSC bei 65°C und anschließendes mehrfaches Waschen in 0,1 × SSC bei 65°C für insgesamt etwa 1 Stunde entsprechen; oder die in SEQ ID Nr.: 3 gezeigte Aminosäuresequenz oder eine Aminosäuresequenz umfasst, die zumindest 80% identisch zu SEQ ID Nr.:3 ist. Wie oben bereits erwähnt, umfasst die Sorghumhirsepflanze in Sinne der vorliegenden Erfindung Pflanzen jeglicher Art der Gattung Sorghum, insbesondere der Arten *Sorghum bicolor, Sorghum sudanense* und *Sorghum bicolor* × *Sorghum sudanense* bzw. deren Hybriden sowie sämtliche sich davon ableitenden Sorten. Dementsprechend ist vernünftigerweise anzunehmen, dass sich im Laufe der Artbildung sowie der Sortenzüchtung die Nukleotidsequenz und entsprechend auch die Aminosäuresequenz der Patatin-Phospholipase verändert haben. In diesem Zusammenhang bedeutet der Begriff homolog, dass die betreffenden Gene (aus zwei verschiedenen Pflanzenarten bzw. Sorten) im Wesentlichen die gleiche Funktion und einen gemeinsamen Vorläufer haben, und sich daher typischerweise eine signifikante Identität in deren Nukleinsäure- bzw. kodierten Aminosäuresequenz zeigt, welche vorzugsweise bei mindestens 80% liegt.

Im Sinne der Beschreibung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, unter einem "Ortholog" ein Protein aus einer anderen Spezies verstanden, das die gleiche Funktion ausübt und unter einem "Paralog" ein Protein verstanden, das durch Duplikation innerhalb einer Spezies entstanden ist, wobei diese Kopie entweder die gleiche Proteinfunktion behält, ihr Expressionsmuster verändert, jedoch nicht die Funktion, ihre Proteinfunktion ändert oder sich die ursprüngliche Genfunktion zwischen beiden Kopien aufteilt.

Ein kodiertes Protein (bzw. Aminosäuresequenz) ist grundsätzlich dann ein Homolog im Sinne der vorliegenden Beschreibung, wenn es die gleiche Funktion ausübt, unabhängig davon ob es denselben oder einen anderen phylogenetischen Ursprung hat oder aus derselben oder einer anderen Spezies stammt. Eine Homolog ist ferner in der Lage ist, die Eigenschaft der Haploideninduktion zu komplementieren, d.h. durch eine Modifizierung des durch das Homolog kodierenden Genprodukts der Pflanze, aus der das Gen stammt, die Eigenschaft eines Haploideninduktors zu vermitteln oder die Induktionsleistung eines Haploideninduktors zu erhöhen. Dementsprechend kann das betreffende Homolog zur Patatin-Phospholipase kodiert durch die Nukleotidsequenz gemäß SEQ ID Nr.: 1 oder 2 bzw. umfassend die Aminosäuresequenz gemäß SEQ ID Nr.: 3 vorzugsweise dadurch gekennzeichnet werden, dass es in der Lage ist die Eigenschaft eines Haploideninduktors, die in der erfindungsgemäßen Sorghumhirsepflanze beobachtet wird, zu komplementieren. Zusätzlich oder alternativ kann das Patatin-Phospholipase-Homolog vorzugsweise dadurch gekennzeichnet werden, dass durch die Modifizierung des durch das Homolog kodierten Genprodukts die Eigenschaft eines Haploideninduktors vermittelt oder die Induktionsleistung eines Haploideninduktors erhöht wird.

Entsprechende Techniken und Verfahren zu Komplementationsgenetik in Sorghum sind dem Fachmann aus dem Stand der Technik bekannt, beispielsweise aus der Publikation von Li et al., J Genet. 94 (2015), 445-452, in der der Sorghum-Phänotyp, der sich durch eine braune Mittelrippe auszeichnet und durch eine Punktmutation in dem *bmr-6* Gen verursacht wird, durch Einbringen des Wildtpy-bmr-6 Gens komplementiert wurde.

Wie in Beispiel 2 gezeigt, führt ein Aminosäureaustausch in der Aminosäuresequenz der Patatin-Phospholipase aus Sorghumhirse (SEQ ID Nr.: 3) in Sorghumhirse zu einer Haploideninduktionsrate von teilweise mehr als 1,5%. Es ist davon auszugehen, dass durch weitere Aminosäureaustausche, was zu einer weiteren Modifizierung der kodierenden Sequenz der Patatin-Phospholipase führt, es zu einer weiteren Erhöhung der Induktionsrate kommen kann. Somit sind von der vorliegenden Erfindung Sorghumhirsepflanzen umfasst, die eine oder mehrere Modifikationen bzw. Mutationen aufweisen, die die Patatin-Phospholipase betreffen.

Die oben erwähnten Modifikationen, die die Patatin-Phospholipase betreffen, sind im Sinne der vorliegenden Erfindung dadurch gekennzeichnet, dass es Mutationen sind, die in dem endogenen Gen kodierend die Patatin-Phospholipase, zu einem oder mehreren Aminosäureaustauschen in dem Bereich der Aminosäurepositionen 55-75, 157-167 oder 285-298 gemäß SEQ ID Nr.: 3 führen.

Eine Mutation meint eine Modifikation auf DNA-Ebene, also eine Veränderung der Genetik und/oder der Epigenetik. Beispielsweise kann eine Veränderung der Genetik der Austausch von mindestens einer Nukleobase in der endogenen DNA-Sequenz oder in einer regulatorischen Sequenz der endogenen DNA-Sequenz sein. Findet ein solcher Nukleobasen-Austausch z.B. in einem Promotor statt, kann dies zu einer veränderten Aktivität des Promotors führen, da beispielsweise cis-regulatorische Elemente derart modifiziert werden, dass die Affinität eines Transkriptionsfaktors zu dem mutierten *cis*-regulatorischen Element im Vergleich zum Wildtyp-Promotor verändert ist, sodass die Aktivität des Promotors mit dem mutierten *cis-*regulatorischen Element erhöht oder erniedrigt ist, je nachdem ob es sich bei dem Transkriptionsfaktor um einen Repressor oder Induktor handelt oder ob die Affinität des Transkriptionsfaktors zu dem mutierten cis-regulatorischen Element verstärkt oder abgeschwächt wird. Findet ein solcher Nukleobasen-Austausch z.B. in einer kodierenden Region der endogenen DNA-Sequenz statt, kann dies zu einem Aminosäureaustausch im kodierten Protein führen, was eine Veränderung der Aktivität oder Stabilität des Proteins im Vergleich zum Wildtyp-Protein bewirken kann. Ein weiteres Beispiel für eine Veränderung der Genetik ist die Deletion von Nukleotiden in der regulatorische Sequenz und/oder der endogenen DNA-Sequenz sowie die Addition von Nukleotiden in der regulatorischen Sequenz und/oder der endogenen DNA-Sequenz. Eine Veränderung der Epigenetik kann beispielsweise durch ein verändertes Methylierungsmuster der DNA erfolgen.

Verfahren zum Mutagenisieren von DNA-Sequenzen werden im Zusammenhang mit dem Verfahren zum Erhalt eines pflanzlichen Haploideninduktors näher beschrieben.

Da die beschriebenen Mutationen zu einer Änderung oder Verkürzung der Aminosäuresequenz der Patatin-Phospholipase führen, kann davon ausgegangen werden, dass die Mutationen die Aktivität oder Stabilität der durch die endogene DNA-Sequenz kodierten Patatin-Phospholipase in der Sorghumhirsepflanze im Vergleich zu einer Wildtyp-Pflanze verändern, d.h. erhöhen oder reduzieren.

Im Falle der Mutationen kann es zu einer Erhöhung der Aktivität des Proteins kommen, zum Beispiel durch Optimierung der Sequenz oder ebenso gut zu einer Inhibierung oder zum Verlust der Aktivität. Auch kann es durch Mutationen in dem Promotorbereich zu einer Änderung der Expression des Gens kommen.

Bezogen auf die vorliegende Erfindung kann demensprechend durch die beschriebene Mutation die biologische Aktivität der Patatin-Phospholipase dahingehend geändert werden, dass ihre ursprüngliche Funktion in Pollen nicht mehr in dem Maße ausgeübt wird, wie es in der Wildtyp-Sorghumhirsepflanze der Fall ist.

Dies kann einerseits durch Überexpression des Gens verbunden mit einer erhöhten Proteinmenge und Aktivität geschehen, beispielsweise durch Mutationen in dem Promotorbereich, durch die Erhöhung der Stabilität des Proteins, oder durch die Bildung einer aktiveren Form der Patatin-Phospholipase, beispielsweise durch Aminosäuresubstitutionen in der funktionellen Domäne, was beispielsweise zu einem schnelleren Wachstum des Pollenschlauchs, einer Entkopplung des Transports der generativen Zellen im Pollenschlauch mit dessen Wachstum oder einer gestörten Interaktion der Gametophyten gefolgt von einer unvollständigen Befruchtung mit anschließender Chromosomeneliminierung führen könnte. Andererseits könnte auch durch die Überexpression des Gens die korrekte Bildung, Faltung und/oder Stabilität der Patatin-Phospholipase inhibiert, verhindert oder herabgesetzt werden. Es könnte jedoch auch zu einer verringerten Bildung oder keiner Bildung einer funktionalen Patatin-Phospholipase kommen, beispielsweise durch Mutationen in dem Promotorbereich, zu der Bildung einer weniger aktiven, inaktiven oder instabilen Form der Patatin-Phospholipase, beispielsweise durch Aminosäuresubstitutionen in der funktionellen Domäne, was auch wiederum zu einer fehlerhaften Befruchtung führen könnte. Ebenso könnte durch die beschriebenen Mutationen die Lokalisation der Patatin-Phospholipase verändert werden, sodass sie beispielsweise nicht mehr pollenspezifisch exprimiert wird.

Infolgedessen sind in der vorliegenden Erfindung auch Sorghumhirsepflanzen umfasst, die eine oder mehrere Insertionen einer Expressionskassette aufweisen, die ein Nukleinsäuremolekül kodierend die Patatin-Phospholipase, die durch die Nukleotidsequenz gemäß SEQ ID Nr.: 1 oder 2 oder durch eine Nukleotidsequenz, die zu mindestens 80%, vorzugsweise 85%, weiter bevorzugt 90%, noch weiter bevorzugt 95% und am bevorzugtesten 99% identisch ist zu SEQ ID Nr.: 1 oder 2 kodiert wird, oder die ein Nukleinsäuremolekül aufweisend eine Nukleotidsequenz, die mit der komplementären Sequenz zu der Nukleotidsequenz gemäß SEQ ID Nr.: 1 oder 2 unter stringenten Bedingungen hybridisiert, wobei die stringenten Bedingungen Hybridisieren in 4 × SSC bei 65°C und anschließendes mehrfaches Waschen in 0,1 × SSC bei 65°C für insgesamt etwa 1 Stunde entsprechen, oder die ein Nukleinsäuremolekül kodierend die Patatin-Phospholipase mit einer Aminosäuresequenz gemäß der SEQ ID Nr.: 3 oder einer Aminosäuresequenz die zu mindestens 80% identisch ist zu SEQ ID Nr.: 3, wobei die kodierte Patatin-Phospholipase oder der funktionellen Teil davon eine oder mehrere Mutationen aufweist, welche zu einem oder mehreren Aminosäureaustauschen und somit für eine Überexpression des Gens kodierend die Patatin-Phospholipase in einer Pflanze oder einem Teil davon im Vergleich zu einer Wildtyp-Pflanze oder entsprechenden Teil davon sorgt.

Wie in Beispiel 2 gezeigt, führt ein Austausch der Aminosäure Arginin gegen Glutamin an der Aminosäureposition 59 gemäß der Aminosäuresequenz der Patatin-Phospholipase gezeigt in SEQ ID Nr.: 6 oder ein Austausch der Aminosäure Valin durch Isoleucin an der Aminosäureposition 162 gemäß der Aminosäuresequenz der Patatin-Phospholipase gezeigt in SEQ ID Nr.: 9 in Sorghumhirsepflanzen zu einer Haploideninduktionsrate von mehr als 0,5%. Anhand von Fig. 2 ist zu erkennen, dass diese Aminosäurepositionen in der funktionellen Domäne der Patatin-Phospholipase liegen, die dem Bereich der Aminosäurepositionen 37 bis 240 entspricht.

Somit stellt die vorliegende Erfindung Sorghumhirsepflanzen bereit, die eine oder mehrere Mutationen aufweisen, die in dem Bereich der Aminosäurepositionen 55-75 oder 157-167 gemäß SEQ ID Nr.:3 zu einem Aminosäureaustausch führen.

Des Weiteren führt ein Austausch der Aminosäure Serin gegen Leucin an der Aminosäureposition 291 gemäß der Aminosäuresequenz der Patatin-Phospholipase gezeigt in SEQ ID Nr.: 12 in Sorghumhirsepflanzen zu einer Haploideninduktionsrate von mehr als 1,5%.

Anhand von Fig. 2 ist zu erkennen, dass diese Position außerhalb der funktionellen Domäne der Patatin-Phospholipase liegen.

Somit stellt die vorliegende Erfindung auch Sorghumhirsepflanzen bereit, die eine oder mehrere Mutationen aufweisen, die in dem Bereich der Aminosäurepositionen 285-298 gemäß SEQ ID Nr.: 3 zu einem Aminosäureaustausch führen, was vorzugsweise einer Region außerhalb der funktionellen Domäne der Patatin-Phospholipase entspricht.

In einer besonders bevorzugten Ausführungsform der vorliegenden Erfindung ist die erfindungsgemäße Pflanze dadurch gekennzeichnet, dass die eine oder mehrere Mutationen zu einem Aminosäureaustausch an der Aminosäureposition 59, 162 und/oder 291 gemäß SEQ ID Nr.: 3 führen.

In diesem Zusammenhang umfasst in einer Ausführungsform der vorliegenden Erfindung die modifizierte Patatin-Phospholipase eine Aminosäuresequenz gemäß SEQ ID Nr.: 3 oder eine Aminosäuresequenz, die zu mindestens 80% identisch ist zu SEQ ID Nr.: 3, in der mindestens ein Aminosäureaustausch vorhanden ist, wobei an der Position 59 Arginin (R), an der Position 162 Valin (V), und/oder an der Position 291 Serin (S) durch eine andere Aminosäure ausgetauscht ist, vorzugsweise durch Glutamin (Q) an der Position 59, Isoleucin (I) an der Position 162 und/oder Leucin (L) an der Position 291; beziehungsweise wird durch eine Nukleotidsequenz gemäß SEQ ID Nr.: 1 kodiert die die kodierende Sequenz der DNA-Sequenz gemäß SEQ ID Nr.: 1 oder einer DNA-Sequenz, die zu mindestens 80% identisch ist zu SEQ ID Nr.: 1 umfasst, in der mindestens ein Nukleotidaustausch vorhanden ist, was zu einem Aminosäureaustauch, wobei an den Positionen 421-423, 815-817, 1420-1422 und/oder 1663-1665 gemäß SEQ ID Nr.: 1 (entsprechend der Nukleotid-Positionen 175-177, 484-486, 871-873 und/oder 1114-1116 der SEQ ID Nr.: 2) ein oder mehrere Nukleotide ausgetauscht sind; oder eine Aminosäuresequenz gemäß SEQ ID Nr.: 6, 9 oder 12; oder durch eine Nukleotidsequenz kodiert wird, die die kodierende Sequenz der DNA-Sequenz gemäß SEQ ID Nr.: 4, SEQ ID Nr.: 7, SEQ ID Nr.: 10 oder SEQ ID Nr.: 13 umfasst.

Pflanzen besitzen als Eukaryoten zwei oder mehr Kopien ihrer genetischen Information pro Zelle. Jedes Gen wird in der Regel durch zwei Allele, die im homozygoten Zustand identisch bzw. im heterozygoten Zustand unterschiedlich sein können, repräsentiert. Der Phänotyp der erfindungsgemäßen Pflanze wird durch eine oder mehrere Mutationen in der Patatin-Phospholipase verursacht, wobei die Pflanze homozygot oder heterozygot, vorzugsweise homozygot für die mutierte Patatin-Phospholipase ist.

In einer weiteren Ausführungsform der vorliegenden Erfindung wird ein Nukleinsäuremolekül beansprucht, welches die zuvor definierten spezifischen Mutationen umfasst, die zu den Aminosäureaustauschen R59Q, V162I, und/oder S291L basierend auf der Aminosäuresequenz von SEQ ID Nr.: 3 führen. Dabei ist das erfindungsgemäße Nukleinsäuremolekül dadurch gekennzeichnet, dass das Vorhandensein des erfindungsgemäßen Nukleinsäuremoleküls in Abwesenheit einer Wildtyp-Patatin-Phospholipase in einer Pflanze dazu führt, dass die Pflanze in der Lage ist Haploidie zu induzieren oder dass die Induktionsleistung einer Pflanze, die bereits zur Haploideninduktion fähig ist, verbessert wird.

In einer bevorzugten Ausgestaltung der vorliegenden Beschreibung hat die erfindungsgemäße Nukleinsäure oder eine von der Nukleinsäure kodierte RNA oder ein von der Nukleinsäure kodiertes Protein oder Polypeptid Einfluss auf das Pollenschlauch-Wachstum in einer Pflanze, auf die Interaktion zwischen die Gametophyten oder auf die Befruchtung als solches.

DNA-Hybridisierungssonden, die von der modifizierten Sequenz der Patatin-Phospholipase abgeleitet sind, d.h. eine der oben beschriebenen Mutationen umfassen, können zum Identifizieren von erfindungsgemäßen Pflanzen verwendet werden, d.h. zur Detektion der Mutationen im Gen der Patatin-Phospholipase eingesetzt werden. Um eine spezifische Hybridisierung zu erzielen, sollten solche Sonden spezifisch sein und mindestens eine Länge von 15 Nukleotiden, bevorzugt mindestens 20 Nukleotide aufweisen. Eine ausführliche Anleitung zu der Hybridisierung von Nukleinsäuren kann man in Tijssen, Laboratory Techniques in Biochemistry and Molecular Biology - Hybridization with Nucleic Acid Probes, Teil 1, Kapitel 2, "Overview of principles of hybridization and the strategy of nucleic acid probe assays," Elsevier, New York (1993); und in Current Protocols in Molecular Biology, Kapitel 2, Ausubel, et al., eds, Greene Publishing and Wiley Interscience, New York (1995) finden. Die Sonden können auch zum Amplifizieren eines Bereiches der modifizierten Sequenz der Patatin-Phospholipase, der mindestens eine der zuvor beschriebenen Mutationen erhält, durch den bekannten Prozess der Polymerase Kettenreaktion (PCR) eingesetzt werden.

Daher ist ein Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und insbesondere bevorzugt mindestens 100, 200, 300, 500 oder 1000 Nukleotiden Länge Gegenstand der vorliegenden Erfindung, wobei dieses Nukleinsäuremolekül spezifisch an eine zuvor beschriebene Nukleotidsequenz, die das modifizierte Patatin-Phospholipase Gen umfasst, hybridisiert, und eine der Mutationen umfasst oder ein Paar von Nukleinsäuremolekülen, das geeignet ist einen Bereich, der mindestens eine der Mutationen enthält, in einer Polymerase-Kettenreaktion (PCR) zu amplifizieren, vorzugsweise in Form eines Oligonukleotids, vorzugsweise mit einer Länge von maximal 50 Nukleotiden, vorzugsweise eine der folgenden Nukleotidsequenzen gemäß SEQ ID Nr.: 28-30, 32-34, 36-38 oder 40-42 aufweist.

Ein weiterer Gegenstand der Erfindung sind Vektoren, die das erfindungsgemäße Nukleinsäuremolekül umfassen. Ein erfindungsgemäßer Vektor kann das mutierte Gen der Patatin-Phospholipase mit den zuvor erwähnten Eigenschaften der Nukleotidsequenz umfassen, das operativ mit einem heterologen Promotor verknüpft ist oder es kann das mutierte Gen der Patatin-Phospholipase zusammen mit seinem natürlichen Promotor umfassen.

Ein anderer Vektor kann das Wildtyp-Gen der Patatin-Phospholipase umfassen, das operativ mit einem heterologen Promotor verknüpft ist. Des Weiteren kann ein Vektor ein rekombinantes DNA-Molekül enthalten, das eine Nukleotidsequenz aufweist, die für eine doppelsträngige RNA kodiert und somit nach Expression in einer Pflanzenzelle zur Inhibierung der Expression des Patatin-Phospholipase Gens führt.

Weiterhin kann ein Vektor das zuvor beschriebene Nukleinsäuremolekül enthalten, das spezifisch an die mutierte Nukleotidsequenz der Patatin-Phospholipase bindet.

Bei dem beschriebenen Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Nukleinsäuremolekül in einem Expressionsvektor mit einer oder mehreren regulatorischen Sequenzen, welche die Transkription und optional die Expression in einer prokaryotischen oder eukaryotischen Wirtszelle erlauben, operativ verknüpft; siehe beispielsweise Sambrook et al., Molecular Cloning: A Laboratory Manual 3rd Ed. Cold Spring Harbor Laboratory Press, Cold Spring Harbor, NY, 2001 und die internationale Anmeldung WO 00/75359 auf Seite 21, Zeile 20 bis Seite 22, Zeile 32. Eine regulatorische Sequenz, vorzugsweise DNA, kann homolog oder heterolog zu der erfindungsgemäßen Nukleinsäure sein. Vorzugsweise sind diese regulatorischen Sequenzen Promotoren oder Terminatoren insbesondere ein Transkriptions-Initiations-Startpunkt, eine Ribosomen-Bindestelle, ein RNA-prozessierendes Signal, eine Transkriptions-Terminations Stelle und/oder ein Polyadenylierungssignal. Die Vektoren enthalten zusätzlich für gewöhnlich Indikator-/Reportergene oder Resistenzgene zum Nachweisen der Übertragung des gewünschten Vektors bzw. DNA-Moleküls/Nukleinsäuremoleküls und zum Selektieren der Individuen die diese enthalten, da ein direkter Nachweis über die Expression des Gens meinst eher schwierig ist. In einer bevorzugten Ausführungsform ist der Vektor ein Pflanzenvektor.

Zusätzlich zu den oben beschriebenen Vektoren, ist ein Verfahren beschrieben, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook *et al.* 2001).

In einem weiteren Aspekt betrifft die vorliegende Erfindung Wirtszellen, die die beschriebenen Vektoren oder Nukleinsäuremoleküle enthalten. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche die erfindungsgemäße Nukleinsäure oder den Vektor umfasst. Dem Fachmann sind sowohl zahlreiche Methoden wie Konjugation oder Elektroporation bekannt, mit denen er die erfindungsgemäße Nukleinsäure oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er die erfindungsgemäße Nukleinsäure oder den Vektor der vorliegenden Erfindung in eine Pflanzenzelle einbringen kann (Sambrook *et al.* 2001).

Weiter bevorzugt betrifft die vorliegende Erfindung eine transgene Pflanzenzelle, die das erfindungsgemäße Nukleinsäuremolekül als Transgen oder den Vektor der vorliegenden Erfindung umfasst sowie eine transgene Pflanze oder einen Teil davon, welche die transgene Pflanzenzelle umfasst. Eine solche transgene Pflanzenzelle oder Pflanze ist beispielsweise eine Pflanzenzelle oder Pflanze, welche mit dem erfindungsgemäßen Nukleinsäuremolekül oder mit dem Vektor der vorliegenden Erfindung vorzugsweise stabil, transformiert ist. Eine transgene Pflanze der vorliegenden Erfindung ist zur Verwendung als Haploideninduktor geeignet. In einer bevorzugten Ausgestaltung der transgenen Pflanzenzelle ist das Nukleinsäuremolekül mit einer oder mehreren regulatorischen Sequenzen, welche die Transkription und optional die Expression in der Pflanzenzelle erlauben, operativ verknüpft. Eine regulatorische Sequenz, vorzugsweise DNA, kann homolog oder heterolog zu der erfindungsgemäßen Nukleinsäure sein. Das Gesamtkonstrukt aus dem erfindungsgemäßen Nukleinsäuremolekül und der/den regulatorischen Sequenzen stellt dann das Transgen dar. Ein Teil einer Pflanze kann ein befruchteter oder unbefruchteter Samen, ein Embryo, ein Pollen, ein Gewebe, ein Organ oder eine pflanzliche Zelle sein, wobei der befruchtete oder unbefruchtete Samen, der Embryo oder der Pollen auf der transgenen Pflanze erzeugt werden und in deren Genom die erfindungsgemäße Nukleinsäure als Transgen oder der Vektor integriert ist. Ebenso schließt die vorliegende Erfindung auch einen Nachkommen der transgenen Pflanze ein, in dessen Genom die erfindungsgemäße Nukleinsäure als Transgen oder der Vektor integriert ist und der zur Verwendung als Haploideninduktor geeignet ist.

In einem weiteren Aspekt betrifft die vorliegende Erfindung ein Verfahren zum Erhalt einer Pflanze, welche in der Lage ist Haploidie zu induzieren bzw. mit einer erhöhten Induktionsrate gegenüber dem Wild-Typ. Das Verfahren umfasst die folgenden Schritte:
(a)
   (i) Mutagenisieren von Pflanzenzellen und anschließendem Regenerieren von Pflanzen aus den mutagenisierten Pflanzenzellen oder Mutagenisieren von Pflanzen; und
   (ii) Identifizieren einer Pflanze aus (i), die eine oder mehrere Mutationen in einer endogenen DNA-Sequenz kodierend die oben beschriebene Patatin-Phospholipase aufweist, die zu einem oder mehreren Aminosäureaustauschen führen, zu einem oder mehreren der beschriebenen Aminosäureaustauschen S291L, R59Q und V1621 gemäß der Aminosäuresequenz SEQ ID Nr. 3 führen oder die zu einem oder mehreren Aminosäureaustauschen in Sequenzen von pflanzlichen Patatin-Phospholipasen führen, die den Aminosäureaustauschen S291L, R59Q und V1621 gemäß der Aminosäuresequenz SEQ ID Nr. 3 entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) in Position 75, Arginin (R) an Position 79 und/oder Leucin (L) an Position 203 oder welche diesen entsprechen, und die in der Lage ist, den Erhalt haploider Nachkommen mit einer erhöhten Rate im Vergleich mit einer nicht-mutagenisierten Pflanze zu induzieren. Dabei verursacht mindestens eine Mutation, dass in der identifizierten Pflanze die Eigenschaft eines Haploideninduktors vermittelt wird oder die Induktionsleistung eines Haploideninduktors erhöht wird, oder
(b)
   (i) Einbringen des Nukleinsäuremoleküls, das eine oder mehrere Mutationen aufweist, welche der einen oder mehreren der in Anspruch 1 gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, in eine Pflanzenzelle, und
   (ii) Regenerieren einer Pflanze aus der Pflanzenzelle aus (i).

Die endogene DNA-Sequenz aus Schritt (a) (ii) des vorstehenden Verfahrens kann grundsätzlich für jedwede pflanzliche Patatin-Phospholipase kodieren. Vorzugsweise kodiert die endogene DNA-Sequenz für eine Patatin-Phospholipase aus Sorghumhirse (SEQ ID Nr.: 3), Sonnenblume (SEQ ID Nr.: 18), Gerste (SEQ ID Nr.: 21), oder Zuckerrübe, wobei Zuckerrübe zwei putative Patatin-Phospholipasen aufweist (SEQ ID Nr.: 24 und 27). Die in dem Schritt (ii) genannten pflanzlichen Patatin-Phospholipasen sind vorzugsweise solche aus Sonnenblume (SEQ ID Nr.: 18), Gerste (SEQ ID Nr.: 21) oder Zuckerrübe, wobei Zuckerrübe zwei putative Patatin-Phospholipasen aufweist (SEQ ID Nr.: 24 und 27).

Dem Fachmann ist bekannt, wie eine Mutation im Sinne der Erfindung durch den Prozess einer Mutagenisierung im Schritt (a) (i) des Verfahrens zur Herstellung einer Pflanze, welche zur Verwendung als Haploideninduktor geeignet ist, erreicht werden kann. Die Mutagenisierung schließt hierbei sowohl die konventionelle Mutagenese als auch die ortspezifische Mutagenese beziehungsweise das "Genome Editing" ein. Bei der konventionellen Mutagenese wird die Modifikation auf DNA-Ebene nicht gezielt herbeigeführt. Die Pflanzenzelle oder die Pflanze wird mutagenen Bedingungen wie z.B. TILLING durch UV-Licht-Bestrahlung oder den Einsatz chemischer Stoffe ausgesetzt (Till et al., BMC Plant Biology 4 (2004), 12). Eine weitere Methode der Zufallsmutagenese ist die Mutagenese mit Hilfe eines Transposons. Die ortspezifische Mutagenese ermöglicht die Einbringung von Modifikation auf DNA-Ebene zielgerichtet an vordefinierten Stellen der DNA. Hierfür können beispielsweise, TALENS (WO 2010/079430, WO 2011/072246), Meganukleasen (Silva et al., Current Gene Therapy 11 (2011), 11), Homing-Endonukleasen (Chevalier, Molecular Cell 10 (2002), 895-905), Zinkfingernukleasen (Lloyd et al., Proceedings of the National Academy of Sciences of the United States of America 102 (2005), 2232-237 oder ein CRISPR/Cas-System (Gaj et al., Trends in Biotechnology 31 (2013), 397-405) verwendet werden.

Das Identifizieren einer Pflanze im Schritt (a) (ii) kann beispielsweise mit Hilfe von molekularen Markern oder Sonden erfolgen. DNA-Sonden sind beispielsweise Primer oder Primerpaare, welche in einer PCR-Reaktion eingesetzt werden können. Beispielsweise können Tillingmutanten durch Sequenzierung des Zielgens in einer Tilling-Population oder weitere Methoden, die Fehlpaarungen in der DNA nachweisen, wie z.B. Schmelzpunktanalysen oder Einsatz fehlpaarungsspezifischer Nukleasen nachgewiesen beziehungsweise identifiziert werden. Vorliegende Erfindung schließt hierfür einsetzbare Primer/Primerpaare ebenfalls mit ein, wie z.B. Primer für die Patatin-Phospholipase.

Ferner können Mutanten, erzeugt mittels Transposons, durch Einsatz von Transposon spezifischen Primern und Zielgen-spezifischem Primern in der PCR über die gesamte Population und anschießende Sequenzierung von PCR-Produkten nachgewiesen werden. Auch solche Primer sind von der vorliegenden Erfindung mit erfasst. Dem Fachmann sind aus dem Stand der Technik weitere Mittel und Verfahren bekannt, welche er zum Identifizieren einer Pflanze im Schritt (b) verwenden kann. Die vorliegende Beschreibung betrifft auch molekulare Marker, welche die Anwesenheit oder Abwesenheit einer Mutation in der endogenen DNA-Sequenz oder in einer regulatorischen Sequenz der endogenen DNA-Sequenz nachweisen. Solche Marker basieren beispielsweise auf einem SNP und sind spezifisch für die Mutation (Beispiele: KASPar oder TaqMan Marker).

Das Identifizieren einer Pflanze im Schritt (a) (ii) kann auch durch Testen der Induktionsleistung wie in dem anhängigen Beispiel 1 beschrieben durchgeführt werden. Somit betrifft die vorliegende Erfindung auch ein Verfahren zur Identifizierung einer erfindungsgemäßen Pflanze durch Nachweis der Mutation in dem Patatin-Phospholipase Gen bzw. durch Nachweis eines Markerallels, das mit der Mutation gekoppelt ist, vorzugsweise unter Verwendung von oben beschriebenen molekulare Marker.

Ein Beispiel für eine Pflanze, die mittels solch einem Verfahren hergestellt und identifiziert wurde, ist die erfindungsgemäße Sorghumhirsepflanze.

Die vorliegende Erfindung betrifft weiterhin auch eine Pflanze, welche mit dem vorstehenden Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die mindestens eine Mutation vorhanden ist. Ebenso schließt die vorliegende Erfindung auch ein Nachkomme der Pflanze ein, welcher die mindestens eine Mutation aufweist und zur Verwendung als Haploideninduktor geeignet ist. Prinzipiell kann das Verfahren auf jedwede Pflanze enthaltend eine Patatin-Phospholipase angewandt werden und dieser somit die Eigenschaft der Haploideninduktion verliehen werden. Vorzugsweise handelt es sich bei dieser Pflanze um Sorghumhirse, Sonnenblume, Gerste, Zuckerrübe, Roggen, Weizen oder Kartoffel.

Das Verfahren zur Herstellung einer transgenen Pflanze, welche zur Verwendung als Haploideninduktor geeignet ist, schließt auch die Bereitstellung von zwei oder mehr der oben beschriebenen Nukleinsäure, wahlweise auch unterschiedliche Ausgestaltungen der erfindungsgemäßen Nukleinsäure und optional in einem oder mehreren Vektoren, und das Transformieren von Pflanzenzellen durch Einbringen der zwei oder mehr Nukleinsäuren ein. Alternativ oder ergänzend können neben der erfindungsgemäßen Nukleinsäure auch eine oder mehrere weitere Nukleinsäuren, welche bekanntermaßen einsetzbar sind zur Erzeugung eines Haploideninduktors (z.B. manipuliertes *cenh3*-Gen (Ravi und Chan, Nature 464 (2010), 615-618; EP 2 989 889 A1; EP 3 037 540 A1; WO 2016/138021 A1) bereitgestellt und transformiert oder züchterisch eingebracht werden.

Das Einbringen des Nukleinsäuremoleküls oder des Vektors aus (b) geschieht mittels Transformation, vorzugsweise mittels stabiler Transformation von Pflanzenzellen. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren sind dem Fachmann geläufig (Sambrook *et al.* 2001). Ein Nukleinsäuremolekül kann zudem auch über homologe Rekombination beispielsweise mittels CRISPR/Cas bzw. CRISPR/Cpf1 und Repairtemplate in das pflanzliche Genom eingebracht werden.

Die vorliegende Erfindung betrifft weiterhin auch eine transgene Pflanze, welche mit diesem Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die eingebrachte Nukleinsäure als Transgen oder der Vektor integriert ist. Ebenso schließt die vorliegende Erfindung auch einen Nachkomme der transgenen Pflanze ein, welcher die eingebrachte Nukleinsäure als Transgen aufweist und zur Verwendung als Haploideninduktor geeignet ist. Prinzipiell kann das Verfahren auf jedwede Pflanze enthaltend eine Patatin-Phospholipase angewandt werden und dieser somit die Eigenschaft der Haploideninduktion verliehen werden. Vorzugsweise handelt es sich bei dieser Pflanze um Sorghumhirse, Sonnenblume, Gerste, Zuckerrübe, Roggen, Weizen oder Kartoffel.

In einem weiteren Aspekt betrifft die vorliegende Beschreibung ein Verfahren zur Herstellung einer haploiden Pflanze, welches die folgenden Schritte umfasst:
(a) Kreuzen einer nicht-transgenen oder transgenen Induktor-Pflanze der vorliegenden Beschreibung, welche zur Verwendung als Haploideninduktor geeignet ist, mit einer Pflanze derselben Gattung, vorzugsweise derselben Spezies,
(b) Selektieren eines befruchtete haploiden Samens oder Embryos, und
(c) Erzeugen einer haploiden Pflanze aus dem Samen oder Embryo aus (b).

Bevorzugt wird die Pflanze, welche zur Verwendung als Haploideninduktor geeignet ist, als Pollenelter eingesetzt und mit einem Saatelter derselben Gattung, vorzugsweise derselben Spezies gekreuzt. Es kann auch die Pflanze, welche zur Verwendung als Haploideninduktor geeignet ist, als Saatelter eingesetzt werden und mit einem Pollenelter derselben Gattung, vorzugsweise derselben Spezies gekreuzt werden. Beide Kreuzungspartner in Schritt (a), also Saatelter und Pollenelter, können auch dasselbe Individuum sein. Dann stellt der Kreuzungsschritt eine Selbstung dar.

Das Selektieren des haploiden befruchteten Samens oder Embryos kann einen Schritt des Nachweises der Haploidie und des Separierens des haploiden befruchteten Samens oder Embryos von polyploiden befruchteten Samen oder Embryos umfassen. Der Nachweis der Haploidie eines befruchteten Samens oder Embryos kann phänotypisch oder genotypisch erfolgen, indem beispielsweise der Induktor mit einem embryo-spezifischen dominanten Marker versehen wird, der in allen diploiden Nachkommen, aber nicht in den induzierten haploiden Nachkommen sichtbar wird. Des Weiteren kann der Ploidie-Status über Durchflusszytometrie bestimmt werden. Außerdem gibt ein vollständig homozygotes Muster von molekularen Markern einen Hinweis auf haploide Pflanzen. Das Separieren kann automatisiert beispielsweise auf Basis von Daten des Nachweises der Haploidie erfolgen.

Die vorliegende Beschreibung betrifft weiterhin auch einen haploiden befruchteten Samen oder Embryo, welcher beim Kreuzen in Schritt (a) des Verfahrens zur Herstellung einer haploiden Pflanze entsteht sowie eine haploide Pflanze, welche mit diesem Verfahren herstellbar ist oder hergestellt ist, oder ein Teil dieser Pflanze, wobei ein Teil einer Pflanze ein Samen, ein Embryo, ein Gewebe, ein Organ oder eine pflanzliche Zelle sein kann. Ebenso schließt die vorliegende Beschreibung auch ein Nachkomme der Pflanze ein.

Weiterhin erfasst die vorliegende Beschreibung auch eine doppelhaploide (diploide) Pflanze oder einen Teil davon, wobei die doppelhaploide (diploide) Pflanze oder der Teil davon durch Chromosomendoppelung der haploiden Pflanze oder des Teils davon erzeugt wurde. Diese doppelhaploiden (diploiden) Pflanzen können durch folgendes Verfahren hergestellt werden:
(a) Herstellen einer haploiden Pflanze mittels dem beschriebenen Verfahrens;
(b) Verdoppeln des haploiden Chromosomensatzes in mindestens einer Zelle der haploiden Pflanze, und
(c) Regenerieren der diploiden Pflanze aus der Zelle aus (b).

In dem beschriebenen Verfahren zur Erzeugung doppelhaploider Pflanzen werden die haploiden Pflanzen aus (a) mit dem Zellteilungshemmstoff Colchizin behandelt. Das führt zur Verdoppelung der Chromosomen. Dem Fachmann ist dieses Verfahren bekannt und es ist beispielsweise in Seguí-Simarro und Nuez, Cytogenetic and Genome Research 120 (2008), 358-369 beschrieben.

Allgemeine Verfahren zur Herstellung von haploiden sowie doppelhaploiden Pflanzen sind dem Fachmann bekannt, beispielsweise aus Dwivedi et al., Biotechnol. Adv. 33 (2015), 812-29 und Murovec & Bohanec, Biochemistry, Genetics and Molecular Biology "Plant Breeding" (2012), eds. Abdurakhmonov, Kapitel 5 und können auf die vorliegende Beschreibung angewandt werden.

Eine weitere Ausführungsform der vorliegenden Beschreibung umfasst ein Verfahren zur Herstellung von hybriden Pflanzen mit den folgenden Schritten:
(a) Kreuzen der beschriebenen doppelhaploiden (diploiden) Pflanze mit einer zweiten Pflanze derselben Gattung, vorzugsweise derselben Spezies,
(b) Selektieren der hybriden Pflanzen hinsichtlich des gewünschten Merkmals.

Die doppelhaploiden Pflanzen sind reinerbig und durch Kreuzen zweier reinerbiger Pflanzen tritt der bekannte Heterosis-Effekt ein, der zu einer besonders ausgeprägte Leistungsfähigkeit von Hybridpflanzen führt. Es sind demensprechend auch die hybride Pflanzen, die durch solch ein Verfahren erhalten wird Gegenstand der vorliegenden Beschreibung.

In einem weiteren Aspekt betrifft die vorliegende Erfindung die Verwendung der erfindungsgemäßen Nukleinsäure oder des erfindungsgemäßen Vektors in einer Pflanze zur Vermittlung der Eigenschaft eines Haploideninduktors oder zur Erhöhung der Induktionsleistung eines Haploideninduktors oder die Verwendung der erfindungsgemäßen Nukleinsäure oder des erfindungsgemäßen Vektors zur Herstellung einer Pflanze oder einer transgenen Pflanze, welche zur Verwendung als Haploideninduktor geeignet ist.

In Experimenten mit Sorghumhirsepflanzen konnte ein Gen aufgefunden werden, im Speziellen das Gen der Patatin-Phospholipase mit einer oder mehreren Mutationen die geeignet waren, der Pflanze die Eigenschaft eines Haploidie-Induktors zu vermitteln sowie eine Induktionsleistung von mindestens 0,4% bis zu 1,5% oder mehr, sodass erstmal ein effizientes und damit wirtschaftlich anwendbares System zur Herstellung haploider und doppelhaploider Sorghumhirsepflanzen für die Hybridzüchtung bereitgestellt werden konnte. Durch das Verfahren zur Herstellung solcher Haploideninduktoren sowie der Identifizierung von Patatin-Phospholipasen in weiteren Kulturpflanzen, kann dieses System ebenso auf diese übertragen werden.

Die folgenden Beispiele erläutern die Erfindung, ohne jedoch den Gegenstand der Erfindung einzuschränken. Sofern nicht anders angegeben, wurden molekularbiologischen Standardmethoden verwendet, siehe beispielsweise (Sambrook et al., Molecular Cloning: A Laboratory Manual 3rd Ed. Cold Spring Harbor Laboratory Press, Cold Spring Harbor, NY, 2001), Fritsch *et al.,* Cold Spring Harbor Laboratory Press: 1989; Mayer et al., Immunochemical Methods In Cell And Molecular Biology, eds., Academic Press, London, 1987) und Weir et al., Handbook Of Experimental Immunology, Volumes I-IV, Blackwell, eds., 1986).

### BEISPIELE

### 1. Identifizierung der Patatin-Phospholipase als Target zur Vermittlung der Eigenschaft der Haploideninduktion in Sorghumhirse

In einer TILLING Population wurde eine Sorghumhirsepflanze gefunden, welche in der Lage war, Haploidie mit einer Effizienz von etwa 1% zu induzieren. Auf der Suche nach der genetischen Grundlage, welche diese Eigenschaft in der identifizierten Sorghumhirsepflanze vermittelt, fokusierte man sich auf verschiedenen Gene als potentielle Targets. Die Auswahl der Gene erfolgte basierend darauf, dass sie vorzugsweise in Fortpflanzungsorganen der Pflanze exprimiert werden und in irgendeiner Art und Weise eine Rolle in der Befruchtung spielen, denn durch fehlerhafte oder unvollständige Befruchtung, beispielsweise durch einen ausbleibenden oder fehlerhaften Transport der generativen Zellen zu den weiblichen Samenanlagen oder durch Einwirkung auf den Energiestoffwechsel des Pollens, kann es zur Chromosomeneliminierung kommen, was zu einem haploiden Chromosomensatz führt.

Nach ersten vorläufigen Untersuchungen im Rahmen der vorliegenden Erfindung wurde dann ein mutiertes Gen gefunden, das mittels der bioinformatischen Methoden BLASTP und Syntenie Studie (Altschul et al., Nucleid Acid Res. 25 (1997), 3389-3402) als Patatin-Phospholipase in Sorghum identifiziert werden konnte, dessen Nukleotid- und Aminosäuresequenz in SEQ ID Nr.: 1 bzw. SEQ ID Nr.: 3 dargestellt sind, und aufgrund von RNA-Sequenzierung (RNASeq) als eine pollenspezifisch exprimierte Patatin-Phospholipase identifiziert wurde. Das mutierte Gen wies eine Punktmutation in der Nukleotidsequenz der Patatin-Phospholipase auf (vgl. SEQ ID Nr.: 10), welche einen Aminosäureaustausch von Serin zu Leucin an Position 291 bewirkte (vgl. SEQ ID Nr.: 12). Die Punktmutation in der Nukleotidsequenz der Patatin-Phospholipase wurde unter Verwendung der PCR-Methode mit den Primern gemäß SEQ ID Nr.: 44 und 45 identifiziert.

Zur Verifikation des mutierten Gens als Ursache für die beobachtete Haploideninduktion wurde der Locus enthaltend das Gen in den genetischen Hintergrund eines Nicht-Induktors durch züchterische und biotechnologische Methoden eingebracht. Hierdurch gelang es, in den Nicht-Induktor die Eigenschaft eines Haploideninduktors mit einer Effizienz von durchschnittlich 1,5%, wenn die Mutation homozygot vorlag, und 1,2%, wenn die Mutation heterozygot vorlag, einzubringen. Während der Verifikation konnte das mutierte Gen mittels PCR mit den Primern gemäß SEQ ID Nr.: 36 bis 38 nachverfolgt werden. Die Induktionsleistung eines potentiellen Induktors wurde durch Bestäubung von Sorghumhirsepflanzen mit den mutierten Sorghumhirsepflanzen (enthaltend das mutierte Gen) durchgeführt. Dabei unterschieden sich die verwendeten Wildtyp-Sorghumhirsepflanzen genetisch über mehrere Marker von der potentiellen Induktorlinie. Diese Marker wurden dazu verwendet, um homozygote Pflanzen zu identifizieren, welche anschließend mittels Durchflusszytometrie auf Haploidie geprüft wurden.

### 2. Erstellung von weiteren in vivo Sorghumhirse-Haploideninduktoren

Nachdem mit dem zuvor beschriebenen Versuch verifiziert wurde, dass die Patatin-Phospholipase in der Sorghumhirse ein geeignetes Target ist, um in der Sorghumhirse die Eigenschaft der Haploideninduktion zu vermitteln, wurden weitere Mutationen in das endogene Wildtyp-Gen eingebracht. Es wurde eine Mutation eingebracht, die zu einem Austausch der Aminosäure Glutamin (Q) gegen ein Stopp-Codon an der Position 372 führte. Dies führte zu einer Verkürzung des Patatin-Phospholipase Proteins und verlieh der Sorghumhirsepflanze enthaltend diese Mutation die Eigenschaft zur Haploideninduktion mit einer Effizienz von durchschnittlich zwischen 1% und 3%. Wie in Fig. 2 dargestellt, liegen die zuvor beschriebenen Mutationen S291L und Q372Stopp außerhalb der funktionellen Domäne der Patatin-Phospholipase. Daher wurden in einem nächsten Versuch Mutationen in das Gen der Patatin-Phospholipase eingebracht, die einerseits einen R59Q- und andererseits einen V162I-Aminosäureaustausch herbeiführten. Diese beiden Mutationen liegen innerhalb der funktionellen Domäne und bewirken, dass die Sorghumhirsepflanze eine Haploideninduktoreigenschaft erhält, wobei erste Versuche ergaben, dass die Induktionsrate über etwa 0,5% liegt. Ein Sequenzalignment der vier Mutanten zusammen mit dem Wildtyp-Protein ist in Fig. 1 dargestellt.

Es ist davon auszugehen, dass zusätzliche oder andere Mutationen bzw. die Kombination von mehreren Mutationen in der Patatin-Phospholipase zu einer erhöhten bzw. weiter erhöhten Induktionsrate führen, sodass weitere Versuche zum Screening von TILLING-Populationen durchgeführt werden können, um eine Pflanze mit erhöhter Induktionsleistung zu identifizieren.

Des Weiteren könnten auch in den verschiedenen Sorghum Arten und davon abgeleiteten Sorten die Mutationen nicht nur mittels TILLING oder anderen Mutageneseverfahren in die Patatin-Phospholipase eingebracht werden sondern es können beispielsweise weitere Haploideninduktoren durch transgene Expression der Patatin-Phospholipase erzeugt werden. Hierfür sind die entsprechenden Gene inklusive ihrer Promotoren aus den Sorghumhirse-Induktorlinien mit den Mutationen S291L, R59Q, V162I und/oder Q372Stopp in der Patatin-Phospholipase gemäß SEQ ID Nr.: 3 zu klonieren. Diese Gene können in einen geeigneten Transformationsvektor kloniert und in die gewünschte Pflanze transformiert werden.

Die Induktionseigenschaft der oben beschriebenen Sorghumhirsepflanzen lässt sich auf die dargestellten Mutationen in der Patatin-Phospholipase zurückführen, was in der Regel zu einer Änderung der biologischen Aktivität dieser führt. Die biologische Aktivität kann jedoch nicht nur durch das Einbringen der beschriebenen Mutationen sondern auch durch zahlreiche weitere gentechnische Verfahren verändert werden.

Beispielsweise kann das Wildtyp-Gen der Patatin-Phospholipase zusammen mit einem geeigneten Promotor in einen Transformationsvektor kloniert und in die gewünschte Pflanze transformiert werden, sodass es zu einer Überexpression der Patatin-Phospholipase kommt. Weiterhin könnte die Patatin-Phospholipase über RNAi in ihrer Aktivität reduziert werden. Hierfür sind beispielsweise Hairpin-Konstrukte herzustellen, welche dann inklusive eines geeigneten Promotors und Terminators, welche eine Transkription des Hairpin-Konstruktes vor oder zum Zeitpunkt der Pollenausbildung erlauben, in einen geeigneten Transformationsvektor kloniert und in die gewünschte Pflanze transformiert werden. Alternativ könnte nach Knockout-Mutanten gesucht werden, die die Aktivität weiter verringern.

### 3. Erstellung von neuen in vivo Haploideninduktoren

Erste Untersuchungen im Rahmen der vorliegenden Erfindung geben begründeten Anlass zu stipulieren, dass in einigen weiteren Kulturpflanzen die Eigenschaft der Haploideninduktion bzw. eine Verbesserung dieser Eigenschaft über Modifikationen der Patatin-Phospholipase vermittelt werden kann, insbesondere über entsprechende, vorstehend für die Sorghumhirsepflanze beschriebenen Mutationen bzw. gemäß vorstehend beschriebenen erfindungsgemäßen Verfahren. Dabei handelt es sich um Target-Gene, die putative Patatin-Phospholipasen kodieren mit einer der folgenden Aminosäuresequenzen in Sonnenblume (SEQ ID Nr.: 18), Gerste (SEQ ID Nr.: 21) und in Zuckerrübe, wobei Zuckerrübe zwei potentielle Phospholipasen aufweist (SEQ ID Nr.: 24 und 27). Ein Sequenzalignment der Proteinsequenzen aus Sorghum, Sonnenblume, Gerste und Zuckerrübe ist in Fig. 2 dargestellt, in dem zusätzlich die putative funktionelle Domäne gekennzeichnet ist **(fett)**. Die Expression dieser Gene in Pollen kann bspw. über RNASeq von Pollen durchgeführt werden.

Gemäß dem vorstehend beschriebenen erfindungsgemäßen Verfahren können nun gezielt Modifikationen betreffend die Patatin-Phospholipase in Sonnenblume, Gerste (und andere Getreide wie Weizen, Roggen und Hafer) und Zuckerrübe eingebracht werden. Beispielsweise können mithilfe des Sequenzalignments in Fig. 2 die Aminosäuren identifiziert werden, die denen an den Positionen 291, 59, 162 und 372 gemäß SEQ ID Nr.: 3 entsprechen und ausgetauscht werden. Dementsprechend sind auch Sonnenblumen-, Gerste- bzw. Getreide- und Zuckerrübenpflanzen Gegenstand der vorliegenden Erfindung, welche in der Lage sind Haploidie zu induzieren, und dadurch gekennzeichnet sind, dass sie eine oder mehrere Modifikationen aufweisen, die eine endogene vorzugsweise pollenspezifisch exprimierte Patatin-Phospholipase betreffen entsprechend eine der der in vorstehenden Items [] oder Ansprüchen insbesondere für Sorghumhirsepflanze gekennzeichneten Ausführungsformen und/oder erhältlich durch das vorstehend beschriebene bzw. in den Ansprüchen gekennzeichnete erfindungsgemäße Verfahren. Es gibt bereits erste Indikationen, dass auch Aminosäureaustausche an Position 75 (Asparaginsäure (D) ist durch Asparagin (N) ersetzt (D75N)), an Position 79 (Glycin (G) ist durch Arginin (R) ersetzt (G79R)) und/oder an Position 203 (Prolin (P) ist durch Leucin (L) ersetzt (P203L)) der Aminosäuresequenz gemäß SEQ ID Nr. 3 sowohl in Sorghum als auch in anderen Kulturarten eine Induktion von Haploiden vermitteln können,

## Patentansprüche

1. Sorghumhirsepflanze, welche in der Lage ist Haploidie zu induzieren, **dadurch gekennzeichnet, dass** die Pflanze eine oder mehrere Modifikationen aufweist, die ein endogenes Gen kodierend eine Patatin-Phospholipase betreffen, wobei die Patatin-Phospholipase durch die Nukleotidsequenz gemäß SEQ ID Nr.: 1 oder 2 oder durch eine Nukleotidsequenz, die zu mindestens 80% identisch ist zu SEQ ID Nr.: 1 oder 2 kodiert wird, oder durch eine Nukleotidsequenz kodiert wird, die mit der komplementären Sequenz zu der Nukleotidsequenz gemäß SEQ ID Nr.: 1 oder 2 unter stringenten Bedingungen hybridisiert, oder die die in SEQ ID Nr.: 3 gezeigte Aminosäuresequenz oder eine Aminosäuresequenz umfasst, die zu mindestens 80% identisch ist zu SEQ ID Nr.: 3, **dadurch gekennzeichnet, dass** die eine oder mehrere Modifikationen eine oder mehrere Mutationen in dem endogenen Gen kodierend die Patatin-Phospholipase sind, welche zu einem oder mehreren Aminosäureaustauschen führen, wobei die eine oder mehrere Mutationen zu einem Aminosäureaustausch in dem Bereich der Aminosäurepositionen 55-75, 157-167 oder 285-298 gemäß SEQ ID Nr.: 3 führen, wobei die stringenten Bedingungen entsprechen Hybridisieren in 4 × SSC bei 65°C und anschließendes mehrfaches Waschen in 0,1 × SSC bei 65°C für insgesamt etwa 1 Stunde.

2. Pflanze nach Anspruch 1, **dadurch gekennzeichnet, dass** die eine oder mehrere Mutationen zu einem Aminosäureaustausch an der Aminosäureposition 59, 162 und/oder 291 gemäß SEQ ID Nr.: 3 führen.

3. Pflanze nach Anspruch 1 oder 2, **dadurch gekennzeichnet, dass** die modifizierte Patatin-Phospholipase
(i) eine Aminosäuresequenz gemäß SEQ ID Nr.: 3 oder eine Aminosäuresequenz umfasst, die zu mindestens 80% identisch ist zu SEQ ID Nr.: 3, in der mindestens ein Aminosäureaustausch vorhanden ist, wobei an der Position 59 Arginin (R), an der Position 162 Valin (V), und/oder an der Position 291 Serin (S) durch eine andere Aminosäure ausgetauscht ist, vorzugsweise durch Glutamin (Q) an Position 59, Isoleucin (I) an Position 162 und/oder Leucin (L) an Position 291;
(ii) durch eine Nukleotidsequenz kodiert wird, die die kodierende Sequenz der DNA-Sequenz gemäß SEQ ID Nr.: 1 oder einer DNA-Sequenz, die zu mindestens 80% identisch ist zu SEQ ID Nr.: 1 umfasst, in der mindestens ein Nukleotidaustausch vorhanden ist, der zu einem Aminosäureaustauch führt, wobei an den Positionen 421-423, 815-817, 1420-1422 und/oder 1663-1665 gemäß SEQ ID Nr.: 1 ein oder mehrere Nukleotide ausgetauscht sind;
(iii) eine Aminosäuresequenz gemäß SEQ ID Nr.: 6, 9 oder 12 umfasst; oder
(iv) durch eine Nukleotidsequenz kodiert wird, die die kodierende Sequenz der DNA-Sequenz gemäß SEQ ID Nr.: 4, SEQ ID Nr.: 7, SEQ ID Nr.: 10 oder SEQ ID Nr.: 13 umfasst.

4. Nukleinsäuremolekül, welches die in Anspruch 1 gekennzeichnete mutierte Patatin-Phospholipase kodiert, welche in einer Pflanze in Abwesenheit einer Wild-Typ Patatin-Phospholipase dazu führt, dass die Pflanze in der Lage ist Haploidie zu induzieren.

5. Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und insbesondere bevorzugt mindestens 100, 200, 300, 500 oder 1000 Nukleotiden Länge, das spezifisch an eine wie in Anspruch 2 und 3 definierte Nukleotidsequenz hybridisiert, und eine der Mutationen umfasst oder ein Paar von Nukleinsäuremolekülen, das geeignet ist einen Bereich, der mindestens eine der Mutationen enthält, in einer Polymerase-Kettenreaktion (PCR) zu amplifizieren, vorzugsweise in Form eines Oligonukleotids, vorzugsweise mit einer Länge von maximal 50 Nukleotiden, das vorzugsweise eine der folgenden Nukleotidsequenzen gemäß SEQ ID Nr.: 28-30, 32-34, 36-38 oder 40-42 aufweist.

6. Wirtszelle, vorzugsweise Pflanzenzelle enthaltend ein Nukleinsäuremolekül nach Anspruch 4 oder 5, einen Vektor, vorzugsweise Pflanzenvektor, umfassend ein Nukleinsäuremolekül nach Anspruch 4 oder 5 oder das Nukleinsäuremolekül nach Anspruch 4 oder 5 als Transgen, optional unter der Kontrolle eines heterologen Promotors.

7. Verfahren zum Erhalt einer Pflanze, welche in der Lage ist Haploidie zu induzieren bzw.
mit einer erhöhten Induktionsrate gegenüber dem Wild-Typ, umfassend die folgenden Schritte:
(a)
(i) Mutagenisieren von Pflanzenzellen und anschließendem Regenerieren von Pflanzen aus den mutagenisierten Pflanzenzellen oder Mutagenisieren von Pflanzen;
(ii) Identifizieren einer Pflanze aus (i), die eine oder mehrere Mutationen in einer endogenen DNA-Sequenz aufweist, welche der einen oder mehreren der in Anspruch 1 gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, und in der Lage ist, den Erhalt haploider Nachkommen mit einer erhöhten Rate im Vergleich mit einer nicht-mutagenisierten Pflanze zu induzieren; oder
(b)
(i) Einbringen des Nukleinsäuremoleküls, das eine oder mehrere Mutationen aufweist, welche der einen oder mehreren der in Anspruch 1 gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, in eine Pflanzenzelle, und
(ii) Regenerieren einer Pflanze aus der Pflanzenzelle aus (i),
vorzugsweise wobei es sich bei der endogenen DNA-Sequenz bzw. dem Nukleinsäuremolekül um eine Nukleotidsequenz handelt, welche die in SEQ ID Nr.: 3 für Sorghumhirse (Sorghum bicolor), SEQ ID Nr.: 18 für Sonnenblume (Helianthus annuus), SEQ ID Nr.: 21 für Gerste (Hordeum vulgare) oder in SEQ ID Nr.: 24 oder 27 für Beta vulgaris (z.B. Zuckerrübe) gezeigten Aminosäuresequenzen kodiert.

8. Pflanze enthaltend Pflanzenzelle nach Anspruch 6 und/oder erhältlich durch ein Verfahren nach Anspruch 7, vorzugsweise wobei es sich bei der Pflanze um Sorghumhirse, Sonnenblume, Roggen, Weizen, Kartoffel, Gerste oder Zuckerrübe handelt.

9. Organ, Pflanzenteil, Gewebe oder Zelle der Pflanze nach einem der Ansprüche 1 bis 3 oder 8 oder Samen oder Nachkommen der Pflanze nach einem der Ansprüche 1 bis 3 oder 8, wobei der Samen oder die Nachkommen die in einem der Ansprüche 1 bis 3 definierte Mutation aufweist und/oder ein Nukleinsäuremolekül nach Anspruch 4 oder 5 oder einen Vektor, vorzugsweise Pflanzenvektor, umfassend ein Nukleinsäuremolekül nach Anspruch 4 oder 5.

## Claims

1. A sorghum millet plant which is capable of inducing haploidy, **characterized in that** the plant has one or more modification(s) concerning an endogenous gene ecoding a patatin phospholipase, wherein the patatin phospholipase is encoded by the nucleotide sequence according to SEQ ID NO: 1 or 2 or by a nucleotide sequence which is at least 80% identical to SEQ ID NO: 1 or 2, or by a nucleotide sequence which hybridizes under stringent conditions with the complementary sequence to the nucleotide sequence according to SEQ ID NO: 1 or 2, or which comprises the amino acid sequence shown in SEQ ID NO: 3 or an amino acid sequence which is at least 80% identical to SEQ ID NO: 3, **characterized in that** the one or more modification(s) are one or more mutation(s) in the endogenous gene encoding the patatin phospholipase which lead to one or more amino acid substitution(s), wherein the one or more mutation(s) lead(s) to an amino acid substitution in the region of amino acid positions 55-75, 157-167 or 285-298 according to SEQ ID NO: 3,
wherein the stringent conditions correspond to hybridization in 4 × SSC at 65°C and subsequent multiple washing in 0.1 × SSC at 65°C for a total of about 1 hour.

2. The plant according to claim 1, **characterized in that** the one or more mutation(s) lead to an amino acid substitution at amino acid position 59, 162 and/or 291 according to SEQ ID NO: 3.

3. The plant according to claim 1 or 2, **characterized in that** the modified patatin phospholipase
(i) comprises an amino acid sequence according to SEQ ID NO: 3 or an amino acid sequence which is at least 80% identical to SEQ ID NO: 3, in which at least one amino acid exchange is present, wherein at position 59 arginine (R), at position 162 valine (V), and/or at position 291 serine (S) is exchanged by another amino acid, preferably by glutamine (Q) at position 59, isoleucine (I) at position 162 and/or leucine (L) at position 291;
(ii) is encoded by a nucleotide sequence comprising the coding sequence of the DNA sequence according to SEQ ID NO: 1 or a DNA sequence which is at least 80% identical to SEQ ID NO: 1, in which at least one nucleotide substitution is present which leads to an amino acid substitution, wherein one or more nucleotides are substituted at positions 421-423, 815-817, 1420-1422 and/or 1663-1665 according to SEQ ID NO: 1;
(iii) comprises an amino acid sequence according to SEQ ID NO: 6, 9 or 12; or
(iv) is encoded by a nucleotide sequence comprising the coding sequence of the DNA sequence according to SEQ ID NO: 4, SEQ ID NO: 7, SEQ ID NO: 10 or SEQ ID NO: 13.

4. A nucleic acid molecule encoding the mutant patatin phospholipase **characterized in** claim 1 which, in a plant in the absence of a wild type patatin phospholipase, leads to the plant being capable of inducing haploidy.

5. A nucleic acid molecule of at least 15, 16, 17, 18, 19 or 20, preferably at least 21, 22, 23, 24 or 25, more preferably at least 30, 35, 40, 45 or 50, and particularly preferably at least 100, 200, 300, 500 or 1000 nucleotides in length, which hybridizes specifically to a nucleotide sequence as defined in claims 2 and 3, and comprising one of said mutations, or a pair of nucleic acid molecules capable of amplifying a region containing at least one of said mutations in a polymerase chain reaction (PCR), preferably in the form of an oligonucleotide, preferably with a maximum length of 50 nucleotides, which preferably has one of the following nucleotide sequences according to SEQ ID NO: 28-30, 32-34, 36-38 or 40-42.

6. A host cell, preferably plant cell containing a nucleic acid molecule according to claim 4 or 5, a vector, preferably plant vector, comprising a nucleic acid molecule according to claim 4 or 5 or the nucleic acid molecule according to claim 4 or 5 as a transgene, optionally under control of a heterologous promoter.

7. A method for obtaining a plant capable of inducing haploidy or having an increased induction rate compared to the wild type, comprising the following steps:
(a)
(i) Mutagenizing plant cells and subsequent regenerating plants from the mutagenized plant cells or mutagenizing plants;
(ii) Identifying a plant from (i) which has one or more mutations in an endogenous DNA sequence corresponding to the one or more mutation(s) **characterized in** claim 1 and/or which lead(s) to aspartic acid (D) at position 75, glycine (G) at position 79, and/or proline (P) at position 203 of the amino acid sequence according to SEQ ID NO: 3 is replaced by another amino acid, preferably by asparagine (N) at position 75, arginine (R) at position 79 and/or leucine (L) at position 203, or which correspond thereto, and is capable of inducing the maintenance of haploid progeny at an increased rate in comparison to a non-mutagenized plant; or
(b)
(i) Inserting the nucleic acid molecule having one or more mutations corresponding to the one or more mutation(s) **characterized in** claim 1 and/or which lead(s) to replacing aspartic acid (D) at position 75, glycine (G) at position 79, and/or proline (P) at position 203 of the amino acid sequence according to SEQ ID NO: 3 is replaced by another amino acid, preferably by asparagine (N) at position 75, arginine (R) at position 79 and/or leucine (L) at position 203 or which correspond thereto, into a plant cell, and
(ii) Regenerating a plant from the plant cell of (i),
preferably wherein the endogenous DNA sequence or nucleic acid molecule is a nucleotide sequence encoding the amino acid sequences presented in SEQ ID NO: 3 for sorghum millet (Sorghum bicolor), SEQ ID NO: 18 for sunflower (Helianthus annuus), SEQ ID NO: 21 for barley (Hordeum vulgare) or in SEQ ID NO: 24 or 27 for Beta vulgaris (e.g. sugar beet).

8. A plant comprising the plant cell according to claim 6 and/or obtainable by the method according to claim 7, preferably wherein the plant is sorghum millet, sunflower, rye, wheat, potato, barley or sugar beet.

9. An organ, a plant part, a tissue or a cell of the plant according to any one of claims 1 to 3 or 8 or a seed or a progeny of the plant according to any one of claims 1 to 3 or 8, wherein the seed or the progeny has the mutation defined in any one of claims 1 to 3 and/or the nucleic acid molecule according to claim 4 or 5 or a vector, preferably a plant vector, comprising the nucleic acid molecule according to claim 4 or 5.

## Revendications

1. Plante de sorgho capable d'induire une haploïdie, **caractérisée en ce que** la plante présente une ou plusieurs modifications qui concerne(nt) un gène endogène codant pour une phospholipase à domaine patatine, la phospholipase à domaine patatine étant codée par la séquence nucléotidique selon la SEQ ID NO : 1 ou 2 ou par une séquence nucléotidique qui est identique à raison d'au moins 80% à la séquence nucléotidique SEQ ID NO : 1 ou 2 ou par une séquence nucléotidique qui s'hybride sous des conditions stringentes avec la séquence complémentaire à la séquence nucléotidique selon SEQ ID NO : 1 ou 2 ou qui comprend la séquence d'acides aminés montrée dans la SEQ ID NO: 3 ou une séquence d'acides aminés qui est identique à raison d'au moins 80% à la SEQ ID NO : 3, **caractérisée en ce que** ladite une ou lesdites plusieurs modifications est/sont une ou plusieurs mutation(s) dans le gène endogène codant pour la phospholipase à domaine patatine qui entraîne(nt) un ou plusieurs échanges d'acides aminés, ladite une ou lesdites plusieurs mutations entraînant un échange d'acides aminés dans la région des positions d'acides aminés 55-75, 157-167 ou 285-298 selon la SEQ ID NO : 3, les conditions stringentes correspondant à une hybridation dans 4 × SSC à 65°C et un lavage multiple consécutif dans 0,1 × SSC à 65°C pendant au total environ 1 heure.

2. Plante selon la revendication 1, **caractérisée en ce que** ladite une ou lesdites plusieurs mutations entraîne(nt) un échange d'acides aminés au niveau de la position d'acide aminé 59, 162 et/ou 291 selon la SEQ ID NO : 3.

3. Plante selon la revendication 1 ou 2, **caractérisée en ce que** la phospholipase à domaine patatine modifiée
(i) comprend une séquence d'acides aminés selon la SEQ ID NO : 3 ou une séquence d'acides aminés qui est identique à raison d'au moins 80% à la SEQ ID NO : 3, dans laquelle il existe au moins un échange d'acide aminé, où, dans la position 59, l'arginine (R), dans la position 162, la valine (V) et/ou, dans la position 291, la sérine (S) est échangée par un autre acide aminé, de préférence par la glutamine (Q) en position 59, l'isoleucine (I) en position 162 et/ou la leucine (L) en position 291 ;
(ii) est codée par une séquence nucléotidique qui comprend la séquence codante de la séquence d'ADN selon la SEQ ID NO : 1 ou d'une séquence d'ADN qui est identique à raison d'au moins 80% à la SEQ ID NO : 1 dans laquelle il existe au moins un échange de nucléotide qui entraîne un échange d'acide aminé, un ou plusieurs nucléotides étant échangé(s) dans les positions 421-423, 815-817, 1420-1422 et/ou 1663-1665 selon la SEQ ID NO : 1 ;
(iii) comprend une séquence d'acides aminés selon les SEQ ID NO : 6, 9 ou 12 ; ou
(iv) est codée par une séquence nucléotidique qui comprend la séquence codante de la séquence d'ADN selon les SEQ ID NO : 4, SEQ ID NO : 7, SEQ ID NO : 10 ou SEQ ID NO : 13.

4. Molécule d'acide nucléique, qui code pour la phospholipase à domaine patatine mutée caractérisée dans la revendication 1, qui entraîne, dans une plante, en l'absence d'une phospholipase à domaine patatine de type sauvage, que la plante est capable d'induire une haploïdie.

5. Molécule d'acide nucléique d'une longueur d'au moins 15, 16, 17, 18, 19 ou 20, de préférence d'au moins 21, 22, 23, 24 ou 25, de manière particulièrement préférée d'au moins 30, 35, 40, 45 ou 50 et en particulier de préférence d'au moins 100, 200, 300, 500 ou 1000 nucléotides, qui s'hybride spécifiquement à une séquence nucléotidique définie dans la revendication 2 et 3 et qui comprend l'une des mutations ou qui contient une paire de molécules d'acide nucléique qui convient pour amplifier une région qui contient au moins l'une des mutations, dans une réaction en chaîne par polymérase (PCR), de préférence sous forme d'un oligonucléotide, présentant de préférence une longueur d'au maximum 50 nucléotides, qui présente de préférence l'une des séquences nucléotidiques suivantes selon les SEQ ID NO : 28-30, 32-34, 36-38 ou 40-42.

6. Cellule hôte, de préférence cellule végétale contenant une molécule d'acide nucléique selon la revendication 4 ou 5, un vecteur, de préférence un vecteur végétal comprenant une molécule d'acide nucléique selon la revendication 4 ou 5 ou la molécule d'acide nucléique selon la revendication 4 ou 5 en tant que transgène, éventuellement sous le contrôle d'un promoteur hétérologue.

7. Procédé destiné à obtenir une plante qui est capable d'induire une haploïdie ou, selon le cas, présentant un taux d'induction augmenté par rapport au type sauvage, comprenant les étapes suivantes :
(a)
(i) mutagénisation de cellules végétales et régénération consécutive de plantes à partir des cellules végétales mutagénisées ou mutagénisation de plantes ;
(ii) identification d'une plante de (i), qui présente une ou plusieurs mutations dans une séquence d'ADN endogène, qui corresponde(nt) à une ou plusieurs des mutations caractérisées dans la revendication 1 et/ou qui entraîne(nt) que, dans la position 75, l'acide aspartique (D), dans la position 79, la glycine (G) et/ou, dans la position 203, la proline (P) de la séquence d'acides aminés selon la SEQ ID NO : 3 est/sont échangée(s) par un autre acide aminé, de préférence par l'asparagine (N) en position 75, l'arginine (R) en position 79 et/ou la leucine (L) en position 203 ou qui correspondent à celles-ci et qui est capable d'induire l'obtention de descendants haploïdes à un taux augmenté par rapport à une plante non mutagénisée ; ou
(b)
(i) introduction de la molécule d'acide nucléique, qui présente une ou plusieurs mutations, qui corresponde(nt) à une ou plusieurs des mutations caractérisées dans la revendication 1 et/ou qui entraîne(nt) que, dans la position 75, l'acide aspartique (D), dans la position 79, la glycine (G) et/ou, dans la position 203, la proline (P) de la séquence d'acides aminés selon la SEQ ID NO : 3 est/sont échangée(s) par un autre acide aminé, de préférence par l'asparagine (N) en position 75, l'arginine (R) en position 79 et/ou la leucine (L) en position 203 ou qui correspondent à celles-ci dans une cellule végétale et
(ii) régénération d'une plante à partir de la cellule végétale de (i), la séquence d'ADN endogène ou, selon le cas, la molécule d'acide nucléique étant de préférence une séquence nucléotidique qui code pour les séquences d'acides aminés montrées dans la SEQ ID NO : 3 pour le sorgho (Sorghum bicolor), dans la SEQ ID NO : 18 pour le tournesol (Helianthus annuus), dans la SEQ ID NO : 21 pour l'orge (Hordeum vulgare) ou dans la SEQ ID NO: 24 ou 27 pour le Beta vulgaris (par exemple la betterave sucrière).

8. Plante contenant une cellule végétale selon la revendication 6 et/ou pouvant être obtenue par un procédé selon la revendication 7, la plante étant de préférence le sorgho, le tournesol, le seigle, le blé, la pomme de terre, l'orge ou la betterave sucrière.

9. Organe, partie de plante, tissu ou cellule de la plante selon l'une des revendications 1 à 3 ou 8 ou graine ou descendant de la plante selon l'une des revendications 1 à 3 ou 8, la graine ou le descendant présentant la mutation définie dans l'une des revendications 1 à 3 et/ou une molécule d'acide nucléique selon la revendication 4 ou 5 ou un vecteur, de préférence un vecteur végétal, comprenant une molécule d'acide nucléique selon la revendication 4 ou 5.
